(19) Europäisches Patentamt
European Patent Office
Office européen des brevets

(11) Veröffentlichungsnummer: **0 005 541**
**A2**

# (12) EUROPÄISCHE PATENTANMELDUNG

(21) Anmeldenummer: **79101496.2**

(22) Anmeldetag: **16.05.79**

(51) Int. Cl.$^2$: **C 07 D 295/06**
C 07 D 211/14, C 07 D 265/30
C 07 D 211/74, C 07 D 295/08
A 61 K 31/445, A 61 K 31/42
A 01 N 9/22, C 07 D 491/10
//C07D295/10

(30) Priorität: **16.05.78 CH 5280/78**

(43) Veröffentlichungstag der Anmeldung:
**28.11.79 Patentblatt 79/24**

(84) Benannte Vertragsstaaten:
**BE CH DE FR GB IT LU NL SE**

(71) Anmelder: **F.Hoffmann-La Roche & Co. Aktiengesellschaft**
**Abt. VIII-Pt**
**CH-4002 Basel(CH)**

(72) Erfinder: **Bohnen, Klaus, Dr.**
**Buchserstrasse 48**
**CH-8157 Dielsdorf(CH)**

(72) Erfinder: **Pfiffner, Albert, Dr.**
**Grampenweg 10**
**CH-8180 Bülach(CH)**

(74) Vertreter: **Lederer, Franz, Dr. et al,**
**Patentanwälte Dr. Franz Lederer Reiner F. Meyer**
**Lucile-Grahn-Strasse 22**
**D-8000 München 80(DE)**

## (54) Substituierte Piperidin-, Morpholin- oder Piperidonderivate, fungizide Mittel, die diese Verbindungen enthalten, Verfahren zu deren Herstellung und Verwendung sowie die Verwendung dieser Verbindungen als Fungizide.

(57) Die Erfindung betrifft heterocyclische Verbindungen der allgemeinen Formel

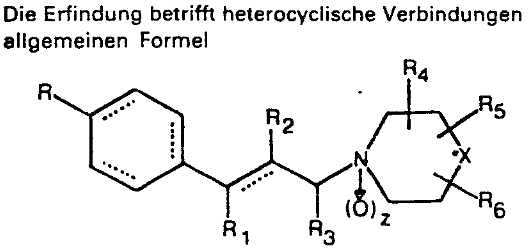

I

worin R Alkyl mit 4-12 Kohlenstoffatomen, wobei der Alkylrest gegebenenfalls mit Halogen substituiert sein kann; Cycloalkyl mit 3-7 Kohlenstoffatomen; Mononieder Alkyl substituiertes Cycloalkyl mit 4-10 Kohlenstoffatomen; Cycloalkylalkyl mit 4-16 Kohlenstoffatomen; Phenyl oder Aryl-nieder Alkyl mit 7-16 Kohlenstoffatomen; $R_1$ Wasserstoff, Hydroxy, Halogen, Alkoxy mit 1-4 Kohlenstoffatomen oder Alkyl mit 1-4 Kohlenstoffatomen; $R_2$ und $R_3$ Wasserstoff oder Alkyl mit 1-4 Kohlenstoffatomen; $R_4$, $R_5$ und $R_6$ Wasserstoff oder Alkyl mit 1-8 Kohlenstoffatomen, wobei zwei der Substituenten $R_4$, $R_5$ und $R_6$ jeweils mit dem gleichen Kohlenstoffatom verknüpft sein können oder zusammen einen ankondensierten alicyclischen oder aromatischen Sechsring bilden können; X eine Methylengruppe, ein Sauerstoffatom, eine Carbonylgruppe oder die Gruppe

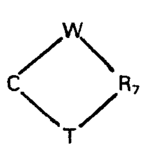

in welcher W und T jeweils für sich ein Sauerstoff- oder Schwefelatom und $R_7$ nieder Alkylen mit 2-4 Kohlenstoffatomen oder Alkenylen mit 4 Kohlenstoffatomen darstellen; oder die Gruppe

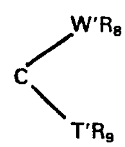

worin W' und T' ein Sauerstoff- oder Schwefelatom und $R_8$ und $R_9$ nieder Alkyl mit 1-6 Kohlenstoffatomen darstellen; Z die ganzen Zahlen 0 oder 1 bedeuten und die gestrichelten Bindungen hydriert sein können; mit der Massgabe, dass im Fall X in der Bedeutung einer Methylengruppe oder eines Sauerstoffatoms vorliegt, entweder der Substituent R lediglich einen Alkylrest mit 4-12 Kohlenstoffatomen bedeutet, der mit Halogen substituiert ist, oder der Substituent $R_1$ in der Bedeutung Hydroxy, Halogen oder Alkoxy mit 1-4 Kohlenstoffatomen vorliegt;
und Salze von solchen Verbindungen, die basischen Charakter aufweisen.

Die Erfindung betrifft ferner ein Verfahren zur Herstellung von Verbindungen der Formel I, fungicide Mittel, welche diese Verbindungen enthalten, Verfahren zur Herstellung dieser Mittel und zu deren Verwendung, sowie die Verwendung dieser Mittel.



Croydon Printing Company Ltd.

0005541

16. Mai 1979

F. Hoffmann-La Roche & Co. Aktiengesellschaft, Basel, Schweiz

RAN 6103/7

# Substituierte Piperidin-, Morpholin- oder Piperidon-derivate, fungizide Mittel, die diese Verbindungen enthalten, Verfahren zu deren Herstellung und Verwendung sowie die Verwendung dieser Verbindungen als Fungizide.

Die Erfindung betrifft heterocyclische Verbindungen der allgemeinen Formel

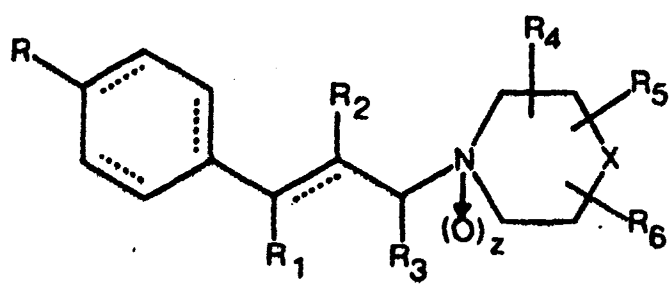

I

worin R Alkyl mit 4-12 Kohlenstoffatomen, wobei der Alkylrest gegebenenfalls mit Halogen substituiert sein kann; Cycloalkyl mit 3-7 Kohlenstoffatomen; Mono-nieder Alkyl substituiertes Cycloalkyl mit 4-10 Kohlenstoffatomen; Cycloalkylalkyl mit 4-16 Kohlenstoffatomen; Phenyl oder Aryl-nieder Alkyl mit 7-16 Kohlenstoffatomen; $R_1$ Wasserstoff, Hydroxy, Halogen, Alkoxy mit

Hof/26.2.1979

1-4 Kohlenstoffatomen oder Alkyl mit 1-4 Kohlenstoffatomen; $R_2$ und $R_3$ Wasserstoff oder Alkyl mit 1-4 Kohlenstoffatomen; $R_4$, $R_5$ und $R_6$ Wasserstoff oder Alkyl mit 1-8 Kohlenstoffatomen, wobei zwei der Substituenten $R_4$, $R_5$ und $R_6$ jeweils mit dem gleichen Kohlenstoffatom verknüpft sein können oder zusammen einen ankondensierten alicyclischen oder aromatischen Sechsring bilden können; X eine Methylengruppe, ein Sauerstoffatom, eine Carbonylgruppe oder die Gruppe

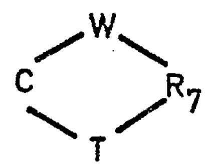

in welcher W und T jeweils für sich ein Sauerstoff- oder Schwefelatom und $R_7$ nieder Alkylen mit 2-4 Kohlenstoffatomen oder Alkenylen mit 4 Kohlenstoffatomen darstellen; oder die Gruppe

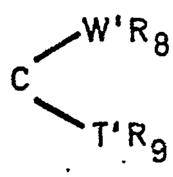

worin W' und T' ein Sauerstoff- oder Schwefelatom und $R_8$ und $R_9$ nieder Alkyl mit 1-6 Kohlenstoffatomen darstellen; Z die ganzen Zahlen O oder 1 bedeuten und die gestrichelten Bindungen hydriert sein können; mit der Massgabe, dass im Fall X in der Bedeutung einer Methylengruppe oder eines Sauerstoffatoms vorliegt, entweder der Substituent R lediglich einen Alkylrest mit 4-12 Kohlenstoffatomen bedeutet, der mit Halogen substituiert ist, oder der Substituent $R_1$ in der Bedeutung Hydroxy, Halogen oder Alkoxy mit 1-4 Kohlenstoffatomen vorliegt;

und Salze von solchen Verbindungen, die basischen Charakter aufweisen.

Soweit nicht anders angegeben umfasst der Ausdruck "nieder Alkyl" Alkylreste mit 1 bis 6 Kohlenstoffatomen wie Methyl, Aethyl, Propyl, Isopropyl, Butyl, Isobutyl und tert.-Butyl.

Alkylreste mit 4 bis 12 Kohlenstoffatomen sind vorzugsweise verzweigte Kohlenwasserstoffreste wie beispielsweise Isobutyl, tert.-Butyl, Neopentyl, 1,1-Dimethylpropyl, 1,1-Dimethylpentyl, 1,1-Diäthylpropyl, 1,1-Dimethylbutyl, 1-Isopropyl-3-methyl-but-1-yl oder 1-Aethyl-1-methyl-butyl. Der Ausdruck Cycloalkylalkyl umfasst auch solche Reste, in welchen sowohl der Cycloalkylteil als auch der Alkylteil durch nieder Alkyl substituiert sein kann.

Der Ausdruck Aryl-nieder Alkyl umfasst sowohl im Arylring mono- oder di-nieder Alkyl als auch im nieder Alkylteil mono- oder di-nieder-Alkyl substituierte Reste wie beispielsweise Benzyl, Phenyläthyl, nieder Alkyl substituiertes Benzyl, wie Methylbenzyl, Dimethylbenzyl oder Naphthylmethyl oder wie beispielsweise 2-Phenyl-propan-2-yl oder 1-phenyl-1-äthyl.

Der Ausdruck Halogen umfasst, wenn nicht anderweitig angegeben, Fluor, Chlor, Brom und Jod.

Verbindungen der Formel I, soweit diese basischen Charakter aufweisen, bilden Salze mit organischen und anorganischen Säuren. Als Salze für die Verbindungen der Formel I kommen insbesondere Salze mit physiologisch verträglichen Säuren in Frage. Hierzu gehören vorzugsweise die Halogenwasserstoffsäuren, wie z.B. die Chlorwasserstoffsäure und die Bromwasserstoffsäure, ferner Phosphorsäure, Salpetersäure, ausserdem mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, und schliesslich Sulfonsäuren, wie die 1,5-Naphthalindisulfonsäure. Die Herstellung von derartigen Salzen erfolgt in an sich bekannter Weise.

Die Erfindung betrifft ferner ein Verfahren zur Herstellung von Verbindungen der Formel I, fungicide Mittel, welche diese Verbindungen enthalten, Verfahren zur Herstellung dieser Mittel und zu deren Verwendung, sowie die Verwendung dieser Mittel.

Das Verfahren zur Herstellung der Verbindungen der Formel I ist dadurch gekennzeichnet, dass man

a) ein Halogenid der Formel

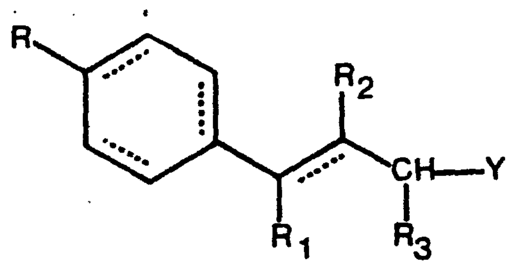

II

worin R, $R_1$, $R_2$, $R_3$ und die gestrichelten Bindungen die in Formel I angegebene Bedeutung besitzen und Y Chlor, Brom oder Jod bedeutet,

mit einer Verbindung der Formel

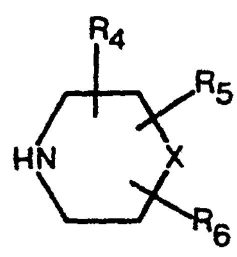

III

worin $R_4$, $R_5$, $R_6$ und X, die in Formel I angegebene Bedeutung besitzen,

umsetzt, oder

b) in einer Verbindung der Formel

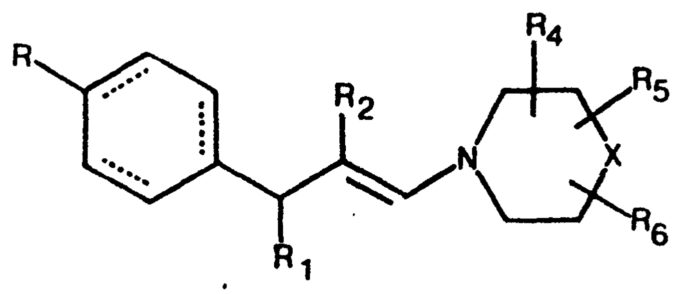

IV

worin R, $R_1$, $R_2$, $R_4$, $R_5$, $R_6$ und X und die gestrichelten Bindungen die in Formel I angegebene Bedeutung besitzen,

die aliphatische Doppelbindung katalytisch hydriert oder mit Ameisensäure reduziert,

c) eine Verbindung der Formel

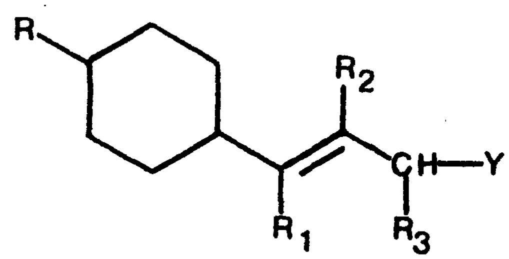

V

worin R, $R_1$, $R_2$, $R_3$ und Y die in Formel II angegebene Bedeutung besitzen,

mit einer Verbindung der Formel III umsetzt, oder

d) eine Verbindung der Formel

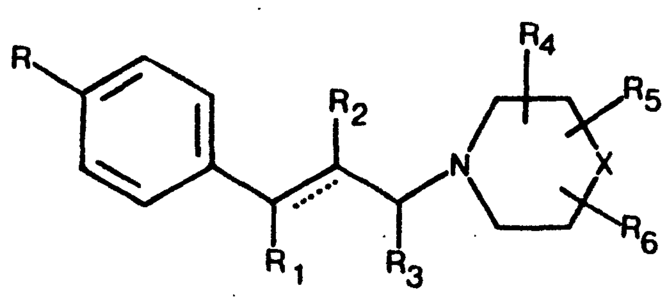

VI

worin R, $R_1$, $R_2$, $R_3$, $R_4$, $R_5$, $R_6$ und X und die gestrichelte Bindung die in Formel I

angegebene Bedeutung besitzen,
katalytisch hydriert, oder

e) eine Verbindung der Formel

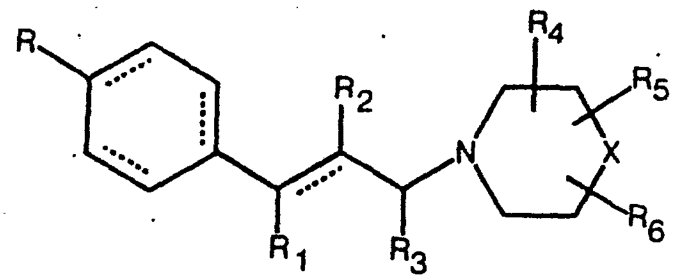

VII

worin R, $R_1$, $R_2$, $R_3$, $R_4$, $R_5$, $R_6$ und X und die gestrichelten Bindungen die in Formel I angegebene Bedeutung besitzen,
mit Wasserstoffperoxid oder Persäuren behandelt, oder

f) eine Verbindung der Formel VII, worin X die Gruppe

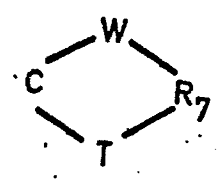

oder die Gruppe

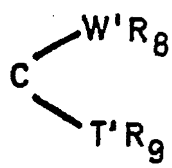

wobei in diesen Gruppen die Symbole W, W', T, T', $R_7$, $R_8$ und $R_9$ die in Formel I angegebene Bedeutung besitzen,
bedeutet, in Gegenwart eines sauren Mittels zu einer Verbindung der Formel VII, worin X eine Carbonylgruppe bedeutet, in an sich bekannter Weise hydrolysiert, oder

g) eine Verbindung der Formel VII, worin X eine Carbonylgruppe bedeutet, in an sich bekannter Weise in die entsprechenden Ketale, Mercaptole, Thioketale oder Hemithioketale der Formel VII, worin X die Gruppe

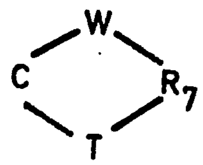

oder die Gruppe 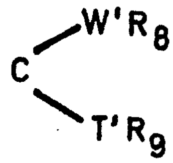

wobei in diesen Gruppen die Symbole W, W', T, T', $R_7$, $R_8$ und $R_9$ die in Formel I angegebene Bedeutung besitzen,

bedeutet, in Gegenwart eines sauren Mittels überführt, oder

h) eine Verbindung der Formel I, die basischen Charakter besitzt, mit einer Säure in an sich bekannter Weise in ein Salz überführt.

Die nachstehend angegebenen römischen Ziffern beziehen sich auf die vorstehend angegebenen Strukturformeln und/oder auf die im nachstehenden Reaktionsschema angegebenen Strukturformeln und/oder auf die bei der nachstehenden Beschreibung der Herstellung der Ausgangsmaterialien angegebenen Strukturformeln. In den Reaktionsschemata A, B und C sind die im Text angegebenen Formeln teilweise aufgeschlüsselt. So umfasst beispielsweise die auf Seite 1 angegebene Formel I alle im Reaktionsschema A angegebenen Formeln mit Ausnahme der Formeln IIa, IIb und IV. In den Reaktionsschemata A, B und C besitzen die Symbole R, $R_1$, $R_2$, $R_3$, $R_4$, $R_5$, $R_6$, X und die gestrichelten Bindungen die in Formel I und Y die in Formel II angegebenen Bedeutungen. Im Reaktionsschema B bedeutet Et den Aethylrest und Ac den Acetylrest. Im Reaktionsschema C bedeutet Hal Halogen und R' Alkyl mit 1-4 Kohlenstoffatomen.

**Reaktionsschema A**

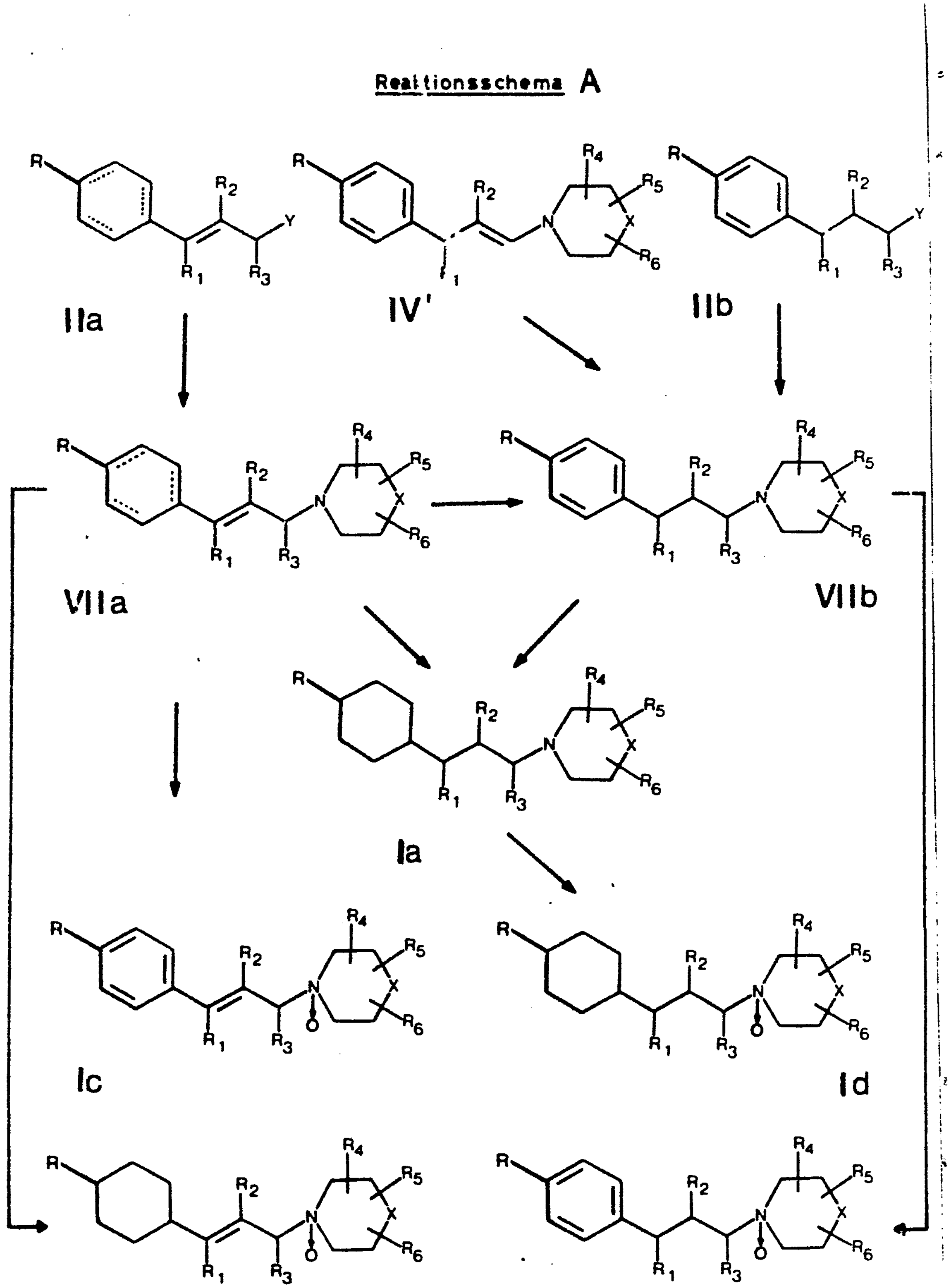

IIa IV' IIb

VIIa VIIb

Ia

Ic Id

Ib Ie

<u>Reaktionsschema</u> B

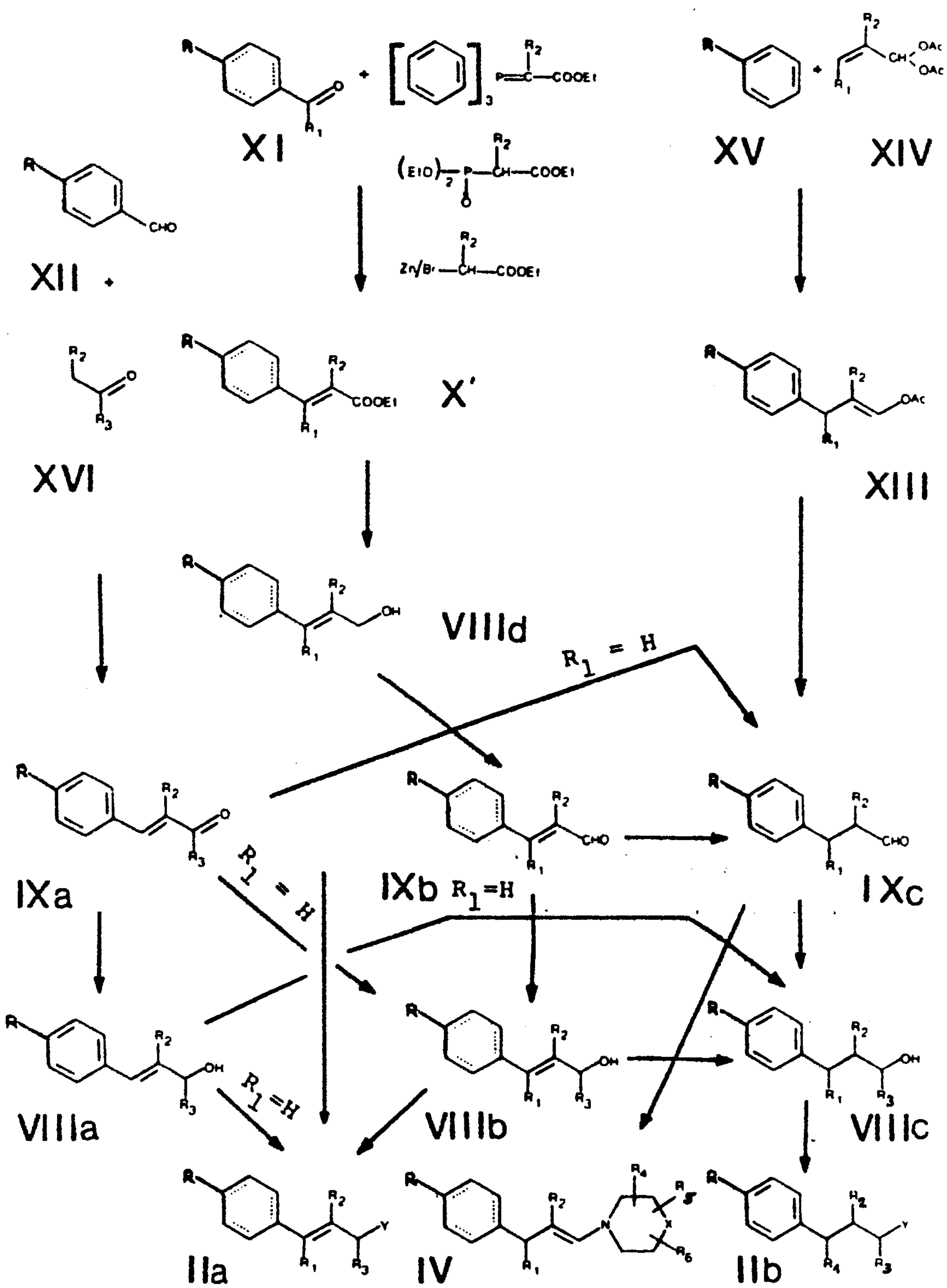

## Reaktionsschema C

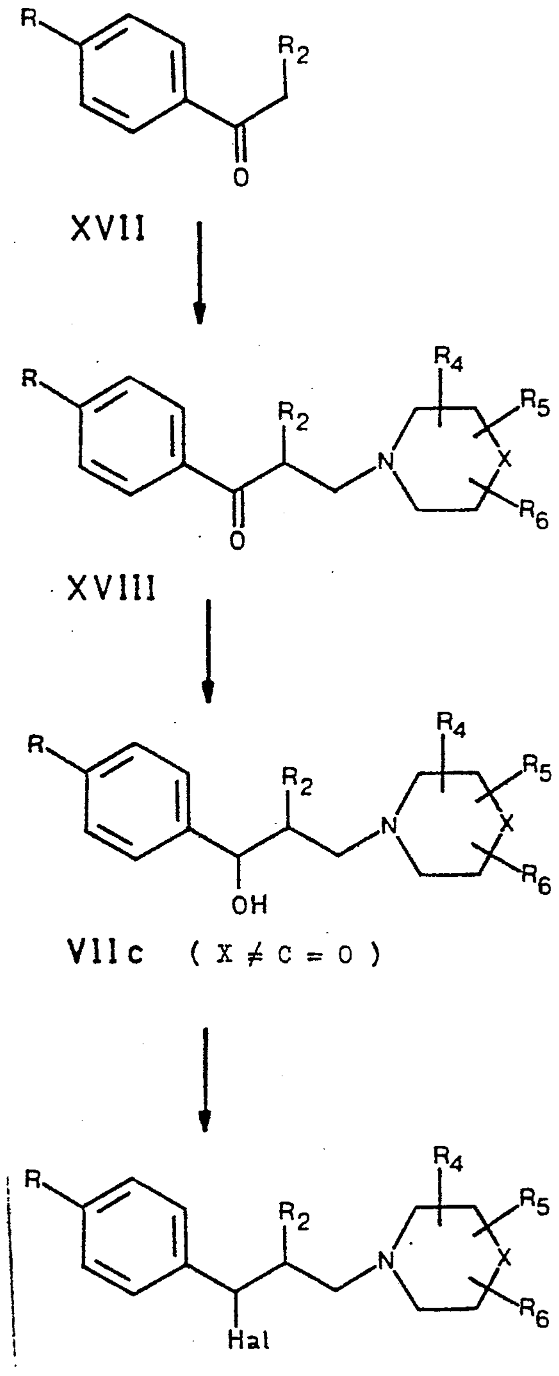

XVII

XVIII

VIIc ( X ≠ C = O )

VIId ( X ≠ C = O )

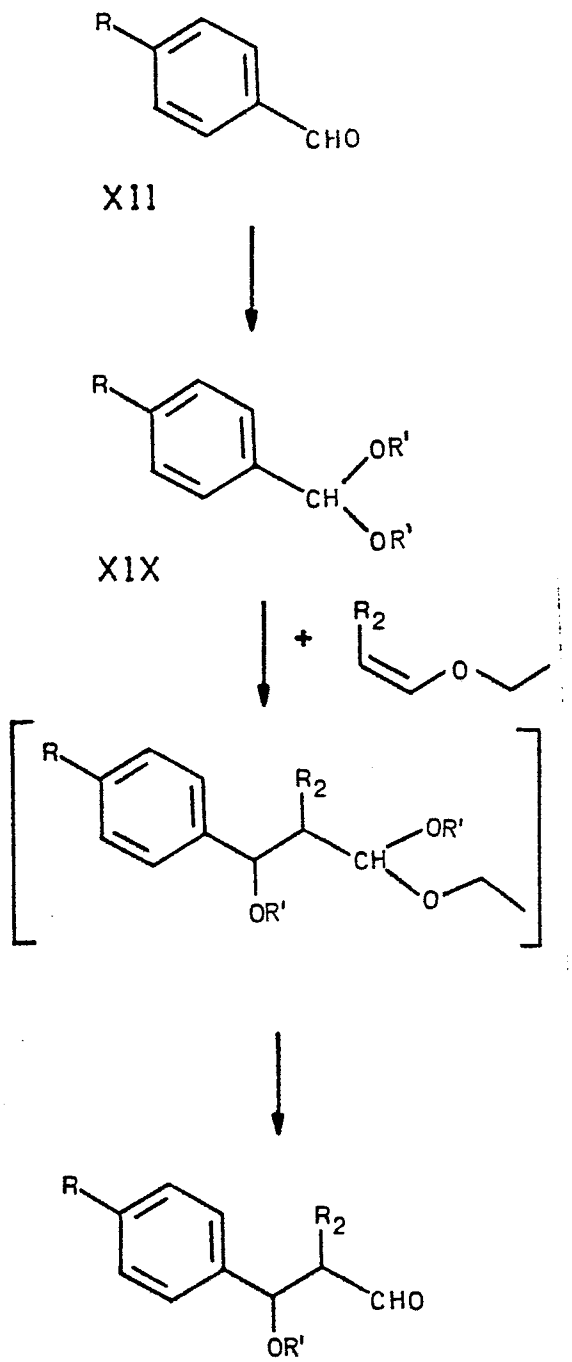

XII

XIX

IXd

Gemäss Verfahrensvariante a) wird ein Halogenid der Formel II mit einem Amin der Formel III in einem inerten Lösungsmittel, vorzugsweise in einem Aether wie Diäthyläther, Tetrahydrofuran oder Dioxan in Gegenwart einer Base wie beispielsweise Triäthylamin oder einem Ueberschuss an Amin der Formel III umgesetzt.

Dient ein Halogenid der Formel IIa als Ausgangsmaterial, so wird vorzugsweise als Lösungsmittel Diäthyläther verwendet. Als Reaktionstemperatur eignet sich besonders ein Intervall zwischen 0°C und Rückflusstemperatur. Bevorzugt ist die Siedetemperatur des Reaktionsgemisches.

Wird die Alkylierung des Amins mit einer Verbindung der Formel IIb durchgeführt, so sind als inerte Lösungsmittel höher siedende Alkohole bevorzugt. Besonders bevorzugt sind Aethylenglykol oder Glycerin. Das Gemisch wird vorzugsweise in einem Temperaturbereich zwischen 50°C und 150°C zur Reaktion gebracht. Besonders bevorzugt ist als Lösungsmittel Aethylenglykol und eine Temperatur von 100-110°C.

Gemäss Verfahrensvariante b) wird eine Verbindung der Formel IV katalytisch hydriert oder mit Ameisensäure reduziert. Als Katalysatoren eignen sich besonders Edelmetallkatalysatoren wie beispielsweise Platin, Palladium- gegebenenfalls auf Kohle niedergeschlagen - sowie Raney-Nickel. Bevorzugt ist Palladium auf Kohle.
Geeignete inerte Lösungsmittel sind Kohlenwasserstoffe wie Benzol, Toluol oder Xylol sowie Alkohole wie Methanol oder Aethanol. Bevorzugt ist Toluol. Als Reaktionstemperatur wird vorteilhaft ein Intervall zwischen 0° und 50°C, bevorzugt Raumtemperatur gewählt.
Die Reduktion des Enamins mit Ameisensäure wird vorzugsweise in Abwesenheit eines Lösungsmittels durchgeführt. Zum Enamin wird bei einer Temperatur von 0° bis 100°C, vorzugsweise 50-70°C, die Ameisensäure, notwendigenfalls unter Kühlung, zugetropft.

Gemäss Verfahrensvariante c) wird eine Verbindung der Formel V unter den vorstehend für Verfahrensvariante a) beschriebenen Bedingungen mit einer Verbindung der Formel III umgesetzt.

Gemäss Verfahrensvariante d) wird eine Verbindung der Formel VI katalytisch hydriert. Als Katalysator dient vorzugsweise Platin oder Palladium, wobei als Lösungsmittel Wasser oder Alkohol zur Verwendung gelangt. Um eine mögliche Hydrogenolyse zu vermeiden, wird dem Reaktionsgemisch mindestens ein Aequivalent Säure, vorzugsweise Salzsäure, zugesetzt. Wird eine Perhydrierung angestrebt, so wird als Katalysator Platin in Eisessig unter Zusatz von Perchlorsäure verwendet. Unter diesen Bedingungen wird der aromatische Rest durchhydriert.

Gemäss Verfahrensvariante e) wird eine Verbindung der Formel VII mit Wasserstoffperoxyd oder einer Persäure behandelt. Wenn eine Verbindung der Formel Ia, VIIa oder VIIb (vgl. Reaktionsschema A) als Ausgangsmaterial dient, wird diese Reaktion mit Wasserstoffperoxyd durchgeführt. Als Lösungsmittel dienen in diesem Fall Alkohole wie Methanol, Aethanol oder Isopropanol, wobei letzterer bevorzugt ist. Bevorzugte Reaktionstemperaturen liegen zwischen 0° und 50°C, besonders bevorzugt bei 40°C.
Dient eine Verbindung der Formel Ia oder VIIb als Ausgangsmaterial, so wird die Reaktion vorzugsweise mit Persäuren wie beispielsweise Peressigsäure, Perbenzoesäure, Metachlorperbenzoesäure, Peradipinsäure usw. oder mit Wasserstoffperoxyd in den entsprechenden Säuren oder Säureanhydriden durchgeführt. Als Lösungsmittel für die Persäuren dienen vorzugsweise halogenierte Kohlenwasserstoffe wie Methylenchlorid, Chloroform oder Aethylenchlorid. Als Reaktionstemperaturen eignen sich die gleichen wie vorstehend für die Reaktion mit Wasserstoffperoxyd beschrieben.

Gemäss Verfahrensvariante f) wird eine Verbindung der Formel VII, worin X die Gruppe

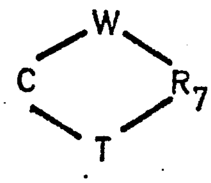

oder

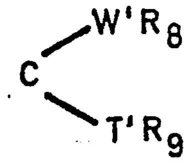

wobei in diesen Gruppen die Symbole W, W', T, T', $R_7$, $R_8$ und $R_9$ die in Formel I angegebene Bedeutung besitzen,

bedeutet, in an sich bekannter Weise mit verdünnten Säuren, vorzugsweise anorganischen Säuren wie Salzsäure, Schwefelsäure oder organischen Säuren wie Ameisensäure oder Essigsäure hydrolysiert.

Gemäss Verfahrensvariante g) wird eine Verbindung der Formel VII, worin X eine Carbonylgruppe bedeutet, mit Diolen, Dithiolen, Mercaptoalkoholen oder Alkoholen und Mercaptanen in Gegenwart eines sauren Mittels wie Zinkchlorid und Natriumsulfat, Bortrifluoridätherat oder p-Toluolsulfonsäure umgesetzt. Die Carbonylgruppe kann jedoch auch ketalisiert werden, indem man die Carbonylverbindung im entsprechenden Alkohol oder Mercaptan löst und beispielsweise gasförmige Salzsäure einleitet.

Eine bevorzugte Gruppe von Verbindungen der Formel I sind solche, worin R in der Bedeutung von 2-Chlor-1,1-dimethyl-äthyl, tert.-Butyl oder tert.-Amyl vorliegt. Eine weitere Gruppe von bevorzugten Verbindungen der Formel I sind solche, worin R der 1-Cyclohexyl-1-methyl-Rest bedeutet.

Bevorzugte Verfahrensendprodukte sind:

1-/3-[p-(2-Chlor-1,1-dimethyl-äthyl)-phenyl]-2-methyl-propyl/-piperidin,

1-/3-[p-(2-Chlor-1,1-dimethyl-äthyl)-phenyl]-2-methyl-propyl/-3,5-dimethyl-piperidin,

4-/3-[p-(2-Chlor-1,1-dimethyl-äthyl)-phenyl]-2-methyl-propyl/-2,6-dimethyl-morpholin,

1-/3-[p-(2-Chlor-1,1-dimethyl-äthyl)-phenyl]-2-methyl-propyl/-4,4-äthylendioxy-piperidin,

1-/3-[p-(2-Chlor-1,1-dimethyl-äthyl)-phenyl]-2-methyl-propyl/-4-piperidon,

1-/3-[p-(2-Chlor-1,1-dimethyl-äthyl)-phenyl]-2,3-dimethyl-2-propenyl/-piperidin,

1-/3-[p-(2-Chlor-1,1-dimethyl-äthyl)-phenyl]-2,3-dimethyl-2-propenyl/-3,5-dimethyl-piperidin,

4-/3-[p-(2-Chlor-1,1-dimethyl-äthyl)-phenyl]-2,3-dimethyl-2-propenyl/-2,6-dimethyl-morpholin,

1-/3-[p-(2-Chlor-1,1-dimethyl-äthyl)-phenyl]-2,3-dimethyl-2-propenyl/-4,4-äthylendioxy-piperidin,

1-/3-[p-(2-Chlor-1,1-dimethyl-äthyl)-phenyl]-2,3-dimethyl-2-propenyl/-4-piperidon,

1-[3-(p-tert.-Butyl-phenyl)-3-hydroxy-2-methyl-propyl]-piperidin,

1-[3-(p-tert.-Butyl-phenyl)-3-methoxy-2-methyl-propyl]-piperidin,

1-[3-(p-tert.-Butyl-phenyl)-3-chlor-2-methyl-propyl]-piperidin,

1-[3-(p-tert.-Butyl-phenyl)-2-methyl-propyl]-4,4-äthylendioxy-piperidin,

1-[3-(p-tert.-Butyl-phenyl)-2-methyl-propyl]-3-methyl-4-piperidon,

1-[3-(p-tert.-Butyl-phenyl)-2-methyl-propyl]-3,5-dimethyl-4-piperidon,

1-[3-(p-tert.-Butyl-phenyl)-2-methyl-propyl]-4-piperidon,

1-[3-(p-tert.-Butyl-phenyl)-2-methyl-propyl]-4,4-äthylendioxy-3-methyl-piperidin,

1-[3-(p-tert.-Butyl-phenyl)-2-methyl-propyl]-4,4-dimethoxy-piperidin,

1-[3-(p-tert.-Butyl-phenyl)-2,3-dimethyl-2-propenyl]-4-piperidon,

1-[3-(p-tert.-Butyl-phenyl)-2,3-dimethyl-2-propenyl]-4,4-äthylendioxy-piperidin,

1-[3-(p-tert.-Amyl-phenyl)-2-methyl-propyl]-4,4-äthylendioxy-piperidin,

1-[3-(p-tert.-Amyl-phenyl)-2-methyl-propyl]-4-piperidon,

1-[3-(p-tert.-Amyl-phenyl)-2-methyl-propyl]-3-methyl-4-piperidon,

1-[3-(p-tert.-Amyl-phenyl)-2-methyl-propyl]-3,5-dimethyl-4-piperidon,

1-[3-(p-tert.-Amyl-phenyl)-2-methyl-propyl]-4,4-äthylendioxy-3-methyl-piperidin,

1-[3-(p-tert.-Amyl-phenyl)-2-methyl-propyl]-4,4-dimethoxy-piperidin,

1-[3-(p-tert.-Amyl-phenyl)-2,3-dimethyl-2-propenyl]-4,4-äthylendioxy-piperidin,

1-[3-(p-tert.-Amyl-phenyl)-2,3-dimethyl-2-propenyl]-4-piperidon,

1-/3-[p-(1-Cyclohexyl-1-methyl)-phenyl]-2-methyl-propyl/-4,4-äthylendioxy-piperidin,

1-/3-[p-(1-Cyclohexyl-1-methyl)-phenyl]-2-methyl-propyl/-4-piperidon,

1-/3-[p-(1-Cyclohexyl-1-methyl)-phenyl]-2,3-dimethyl-2-propenyl/-4,4-äthylendioxy-piperidin,

1-/3-[p-(1-Cyclohexyl-1-methyl)-phenyl]-2,3-dimethyl-2-propenyl/-4-piperidon.

Die Ausgangsmaterialien der Formeln II, IV, V, VI und VII sind teilweise neu.

Die Verbindungen der Formeln VI und VII werden durch Alkylierung eines Amins der Formel III mit einem Halogenid der Formeln II oder V hergestellt. Diese Alkylierung erfolgt gemäss Verfahrensvariante a), die vorstehend beschrieben ist. Die Halogenide können aus dem entsprechenden Alkohol der Formel

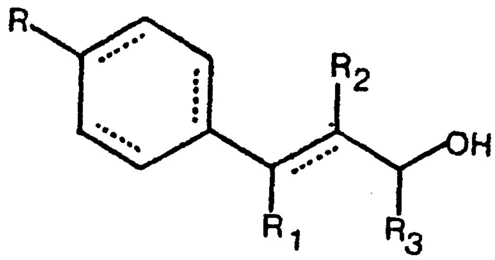

oder

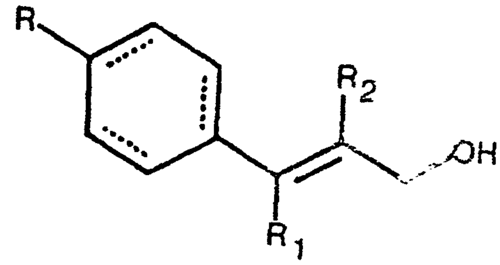

VIII

VIII d

mit einem Phosphorhalogenid wie beispielsweise Phosphortribromid, Phosphortrichlorid, Phosphorpentabromid oder Phosphorpentachlorid mit oder ohne Zusatz einer tertiären Base in an sich bekannter Weise hergestellt werden.
Der Alkohol der Formel VIII oder VIII,d wird aus einer Verbindung der Formel

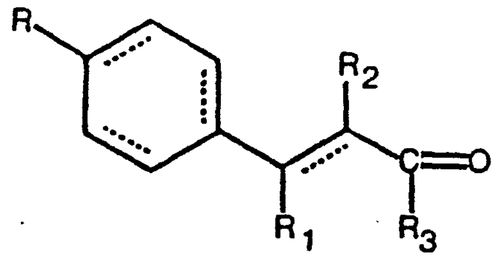

IX

oder

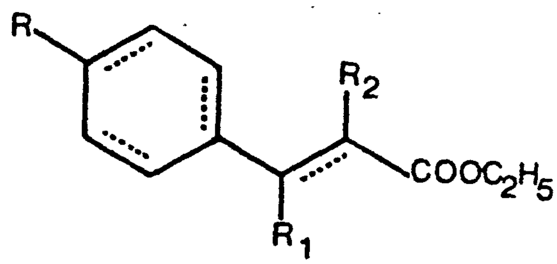

X

durch Reduktion mit einem geeigneten komplexen Hydrid in an sich bekannter Weise erhalten. Geeignete komplexe Hydride zur Reduktion einer Verbindung der Formel IX sind beispielsweise Borhydride wie Natriumborhydrid oder Alanate wie Lithiumaluminiumhydrid. Zur Reduktion einer Verbindung der Formel X ist Lithiumaluminiumhydrid geeignet. Die Verbindungen der Formeln IX und X werden aus dem Aldehyd bzw. Keton der Formel

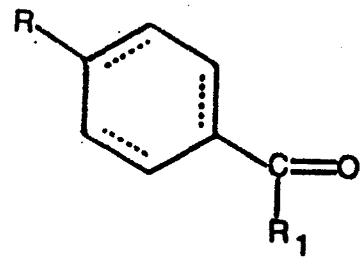

XI

durch eine Wittig-, Horner- oder Reformatzky-Reaktion gewonnen (vgl. Reaktionsschema B).

Als Beispiel für die Wittig- und die Horner-Reaktion wird auf Synthesis (1974), Seite 122ff verwiesen. In dieser Literaturstelle ist auch die einschlägige Sekundärliteratur zitiert. Beispiele für die Reformatzky-Reaktion sind in Bull. Soc. Chim. France (1961), Seite 2145ff beschrieben. In dieser Literaturstelle ist auch eine ausführliche Bibliographie zur Reformatzky-Reaktion angegeben.

Zur Herstellung einer Verbindung der Formel IXa, worin $R_2$ und $R_3$ Alkyl oder $R_2$ Alkyl und $R_3$ Wasserstoff bedeuten, wird der Aldehyd der Formel XII mit dem Keton bzw. Aldehyd der Formel XVI im Sinne einer an sich bekannten Claisen-Schmidt-Kondensation umgesetzt. Einschlägige Literatur ist in "Namenreaktionen der organischen Chemie", Dr. Alfred Hüthig Verlag GmbH, Heidelberg 1961, Seite 94, angegeben.

Eine Verbindung der Formel IXc wird aus einer Verbindung der Formel XIII durch Verseifung in an sich bekannter Weise gewonnen. Die Reaktion wird beispielsweise wie in Bull. Soc. Chim. France (1961), Seite 1194ff beschrieben, durchgeführt. Die Verbindung der Formel XIII wird aus den Verbindungen XV und XIV durch Friedel-Crafts-Reaktion ebenfalls in an sich bekannter Weise hergestellt. Es kann beispielsweise diese Friedel-Crafts-Reaktion in Analogie zu den Beispielen, die in der vorstehend genannten Literaturstelle angegeben sind, durchgeführt werden.

Eine Verbindung der Formel VIIId wird zu einer Verbindung der Formel IXb in an sich bekannter Weise oxydiert.

Beispielsweise können die in J. Org. Chem. 39, 3304 (1974) beschriebenen Methoden zur Anwendung gelangen.

Die Verbindung der Formel Ixb oder IXc kann in an sich bekannter Weise mittels Grignard-Reaktion in die Verbindung der Formel VIIIb oder VIIIc übergeführt werden. Liegt $R_3$ in Verbindung IXa in der Bedeutung Wasserstoff vor, so wird mittels Grignard-Reaktion ebenfalls die Verbindung VIIIb erhalten, worin $R_3$ von Wasserstoff verschieden ist. Bezüglich der Grignard-Reaktion wird auf die Monographie "Grignard Reactions of Nonmetallic Substrates", Verlag Prentice-Hall Inc., New York 1954, verwiesen.

Eine Verbindung der Formel IXa, IXb, VIIIa und VIIIb wird in eine Verbindung der Formel IXc und VIIIc in an sich bekannter Weise übergeführt, indem man das Ausgangsmaterial in einem Alkohol, vorzugsweise Methanol oder Aethanol, gegebenenfalls unter Zusatz von Wasser und wasserlöslichen anorganischen Basen wie beispielsweise Natrium- oder Kaliumcarbonat oder Calciumhydroxid, löst und in Gegenwart von Palladiumkohle bei Raumtemperatur hydriert.

Die Verbindung der Formel IV (vgl. Reaktionsschema B) wird aus einem Aldehyd der Formel IXc durch Umsetzung des Aldehyds mit einer Verbindung der Formel III erhalten. Zu diesem Zweck wird der Aldehyd mit einem Ueberschuss an sekundärem Amin der Formel III versetzt und das Gemisch in Benzol oder Toluol unter Rückfluss erhitzt, wobei das sich bildende Wasser azeotrop abdestilliert wird (vgl. "Advances in Organic Chemistry", Vol. 4, pp. 9ff, Verlag Interscience Publishers, New York, London, 1963).

Die Verbindungen der Formel III, worin X die Gruppe

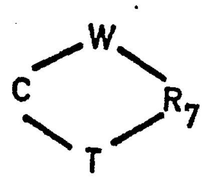 oder die Gruppe 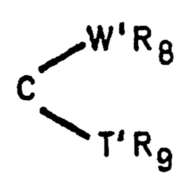

wobei in diesen Gruppen die Symbole W, W', T, T', $R_7$, $R_8$ und $R_9$ die in Formel I angegebene Bedeutung besitzen,

bedeutet, lassen sich aus einer Verbindung der Formel III, worin X eine Carbonylgruppe bedeutet in der Weise wie vorstehend für die Verbindungen der Formel VII beschrieben, herstellen.

Bevorzugte Ausgangsmaterialien der Formel IXb und IXc im erfindungsgemässen Verfahren sind:

p-(2-Chlor-1,1-dimethyl-äthyl)-α,β-dimethyl-zimtaldehyd,

p-tert.-Butyl-α,β-dimethyl-zimtaldehyd,

p-tert.-Amyl-α,β-dimethyl-zimtaldehyd,

p-(1-Cyclohexyl-1-methyl)-α,β-dimethyl-zimtaldehyd,

3-[p-(2-Chlor-1,1-dimethyl-äthyl)-phenyl]-2-methyl-propionaldehyd,

3-(p-tert.-Butyl-phenyl)-2-methyl-propionaldehyd,

3-(p-tert.-Amyl-phenyl)-2-methyl-propionaldehyd,

3-[p-(1-Cyclohexyl-1-methyl)-phenyl]-2-methyl-propionaldehyd,

3-(p-tert.-Butyl-phenyl)-3-methoxy-2-methyl-propionaldehyd.

Bevorzugte Ausgangsmaterialien der Formel IIa im erfindungsgemässen Verfahren sind:

3-[p-(2-Chlor-1,1-dimethyl-äthyl)-phenyl]-2-methyl-allylbromid,

3-[p-(2-Chlor-1,1-dimethyl-äthyl)-phenyl]-2,3-dimethyl-allylbromid,

3-(p-tert.-Butyl-phenyl)-2-methyl-allylbromid,

3-(p-tert.-Butyl-phenyl)-2,3-dimethyl-allylbromid,

3-(p-tert.-Amyl-phenyl)-2-methyl-allylbromid,

3-(p-tert.-Amyl-phenyl)-2,3-dimethyl-allylbromid,

3-(4-tert.-Butyl-cyclohexyl)-2-methyl-allylbromid,

3-(4-tert.-Amyl-cyclohexyl)-2-methyl-allylbromid,

3-[p-(1-Cyclohexyl-1-methyl)-phenyl]-2-methyl-allylbromid,

3-[p-(1-Cyclohexyl-1-methyl)-phenyl]-2,3-dimethyl-allylbromid.

Bevorzugte Ausgangsmaterialien der Formel IIb im erfindungsgemässen Verfahren sind:

3-(p-tert.-Butyl-phenyl)-1,2-dimethyl-propylbromid,
3-(p-tert.-Butyl-phenyl)-1,2,3-trimethyl-propylbromid,
3-(p-tert.-Butyl-phenyl)-2,3-dimethyl-propylbromid,
3-(p-tert.-Amyl-phenyl)-1,2-dimethyl-propylbromid,
3-(p-tert.-Amyl-phenyl)-1,2,3-trimethyl-propylbromid,
3-(p-tert.-Amyl-phenyl)-2,3-dimethyl-propylbromid.

Bevorzugte Ausgangsmaterialien der Formel IV im erfindungsgemässen Verfahren sind:

1-/3-[p-(2-Chlor-1,1-dimethyl-äthyl)-phenyl]-2-methyl-1-propenyl/-piperidin,

1-/3-[p-(2-Chlor-1,1-dimethyl-äthyl)-phenyl]-2-methyl-1-propenyl/-3,5-dimethyl-piperidin,

4-/3-[p-(2-Chlor-1,1-dimethyl-äthyl)-phenyl]-2-methyl-1-propenyl/-2,6-dimethyl-piperidin.

Die Isolierung der Verbindungen der allgemeinen Formel IV ist nicht erforderlich. Sie werden ohne Aufarbeitung entweder durch Zugabe von Ameisensäure, oder durch Hydrierung direkt in Verbindungen der allgemeinen Formel VIIb überführt.

Die Verbindungen der Formel I besitzen fungicide Wirkung und können dementsprechend zur Bekämpfung von Fungi in der Landwirtschaft und im Gartenbau Verwendung finden. Die Verbindungen eignen sich besonders zur Bekämpfung von echten Mehltaupilzen wie beispielsweise Erysiphe graminis (Getreidemehltau), Erysiphe cichoracearum (Gurkenmehltau), Podosphaera leucotricha (Apfelmehltau), Sphaerotheca

pannosa (Rosenmehltau), Oidium tuckeri (echter Rebmehltau); Rostkrankheiten wie beispielsweise solche der Gattungen Puccinia, Uromyces und Hemileia, insbesondere Puccinia graminis (Getreideschwarzrost), Puccinia coronata (Haferkronenrost), Puccinia sorghi (Maisrost), Puccinia striiformis (Getreidegelbrost), Puccinia recondita (Getreidebraunrost), Uromyces fabae und appendiculatus (Buschbohnenroste) sowie gegen Hemileia vastatrix (Kaffeerost) und Phragmidium mucronatum (Rosenrost).

Ferner wirken verschiedene dieser Verbindungen auch gegen folgende phytopathogenen Pilze:

Ustilago avenae (Flugbrand), Venturia inaequalis (Apfelschorf), Cercospora arachidicola (Erdnuss-Blattfleckenkrankheit), Ophiobolus graminis (Getreide-Fusskrankheit), Septoria nodorum (Getreideblatt- und Spelzbräune) oder Marssonina rosae (Rosen-Sternrusstau). Einzelne Substanzen aus dieser Verbindungsklasse besitzen ausgeprägte Nebenwirkungen gegen verschiedene Species folgender Gattungen: Rhizoctonia, Tilletia, Helminthosporium sowie auch teilweise gegen Peronospora, Coniophora, Lenzites, Corticium, Thielaviopsis und Fusarium.

Ausserdem erbringen Verbindungen der Formel I auch Teilwirkungen gegen phythopathogene Bakterien wie beispielsweise Xanthomonas vesicatoria, Xanthomonas oryzae und andere Xanthomonaden sowie auch gegen verschiedene Arten von Erwinia, z.B. Erwinia tracheiphila.

Gewisse Verbindungen der Formel I wirken auch als Insektizide und Akarizide, wobei sich zum Teil auch Insektenwuchsregulatorische Effekte und anti-feedant-Wirkungen zeigen.

Wie aus den nachstehenden biologischen Beispielen hervorgeht, wirken die Verbindungen der Formel I unter Gewächshausbedingungen bereits bei einer Konzentration von 5 mg bis 500 mg Wirksubstanz pro Liter Spritzbrühe. Im Freiland werden vorteilhaft Konzentrationen von 100 g bis 1500 g Wirksubstanz der Formel I pro Hektar und Behandlung zur Anwendung gebracht. Beispielsweise wird zur erfolgreichen Getreidemehltaubekämpfung eine Konzentration von 200 g bis 1000 g, vorzugsweise 200 g bis 600 g Wirksubstanz pro Hektar und Anwendung mit Vorteil benützt. Zur Getreiderostbekämpfung werden vorzugsweise Konzentrationen von 500 g bis 1500 g, besonders bevorzugt hinsichtlich der wirksamsten Vertreter 500 g bis 1000 g Wirksubstanz pro Hektar und Anwendung eingesetzt.

Ein Teil der Verbindungen der Formel I zeichnet sich durch hohe systemische Wirkungsentfaltung aus. Durch Sekundärverteilung der Wirksubstanz (Gasphasenwirkung) können auch nicht behandelte Pflanzenteile geschützt werden.

Für praktische Zwecke können die Verbindungen der Formel I als für Wirbeltiere weitgehend ungiftig qualifiziert werden. Die Toxizität der Verbindungen der Formel I liegt im Durchschnitt oberhalb 1000 mg pro kg Körpergewicht beim akuten Toxizitätstest an der Maus. Einzelne Vertreter zeigen $LD_{50}$-Werte an der Maus zwischen 400 und 1000 mg pro kg Körpergewicht, andere Vertreter zeigen $LD_{50}$-Werte, die zwischen 1000 und 10'000 mg pro kg Körpergewicht im akuten Toxizitätstest an der Maus liegen.

Die nachstehend beschriebenen biologischen Versuche illustrieren die Wirkung der Verbindungen der Formel I und die Resultate sind in den Tabellen zusammengefasst.

a) <u>Erysiphe graminis</u>

30-40 Gerstenkeimlinge der Sorte HERTA (verteilt auf 2 Töpfe mit 7 cm Durchmesser) wurden jeweils im Einblattstadium mit einer wässerigen Dispersion der Testsubstanz

(wie allgemein üblich aufbereitet als Spritzpulver) allseitig gründlich besprüht und anschliessend bei 22-26°C, 80% rel. Luftfeuchtigkeit und einer Photoperiode von 16 Stunden im Gewächshaus weiterkultiviert. Die Infektion erfolgte 2 Tage nach der Behandlung durch Bestäuben der Versuchspflanzen mit Konidien von Erysiphe graminis. 7 Tage nach der Infektion wurde die durch Erysiphe graminis befallene Blattfläche in % gegenüber derjenigen der infizierten, nicht behandelten Kontrolle ermittelt. Die Resultate sind in Tabelle I zusammengefasst.

b) Puccinia coronata

30-40 Haferkeimlinge der Sorte FLAEMINGSKRONE (verteilt auf 2 Töpfe mit 7 cm Durchmesser) wurden jeweils im Einblattstadium mit einer wässerigen Dispersion der Testsubstanz (wie allgemein üblich aufbereitet als Spritzpulver) allseitig gründlich besprüht und anschliessend in einer Klimakabine bei 17°C, 70-80% rel. Luftfeuchtigkeit und einer Photoperiode von 16 Stunden weiterkultiviert. Nach 2 Tagen erfolgte die Infektion der Versuchspflanzen durch Besprühen mit in dest. Wasser suspendierten Uredosporen (300'000 Sporen/ml) von Puccinia coronata. Danach wurden die Pflanzen während 24 Stunden bei 20°C und einer Luftfeuchtigkeit von über 90% im Dunkeln inkubiert und anschliessend in ein Gewächshaus mit einer Temperatur von 22-26°C, einer rel. Luftfeuchtigkeit von 70% und einer Photoperiode von 18 Stunden überführt. Am 9. Tage nach der Infektion wurde die durch Puccinia coronata befallene Blattfläche in % gegenüber der infizierten, nicht behandelten Kontrolle ermittelt. Die Resultate sind in Tabelle I zusammengefasst.

c) Venturia inaequalis

3 Apfelpflänzchen (verteilt auf 3 Töpfe mit 5 cm Durchmesser) aus Samen der Sorte GOLDEN DELICIOUS wurden im 4- bis 5-Blattstadium mit einer wässerigen Dispersion der Testsubstanz (wie allgemein üblich aufbereitet als Spritzpulver) allseitig gründlich besprüht. Die behandelten

Pflanzen wurden anschliessend während 2 Tagen bei 17°C, 70-80%rel. Luftfeuchtigkeit und einer Photoperiode von 14 Stunden weiterkultiviert. Danach erfolgte die Infektion der Apfelsämlinge durch Besprühen mit in dest. Wasser suspendierten Konidien (200'000 Konidien/ml) von Venturia inaequalis. Nach der Infektion wurden die Pflanzen im Dunkeln während 48 Stunden bei 16-18°C und einer rel. Luftfeuchtigkeit von über 90% inkubiert und anschliessend in ein schattiertes Gewächshaus mit einer Temperatur von 22-26°C und einer rel. Luftfeuchtigkeit von über 80% überführt. Am 13. Tage nach der Infektion erfolgte die Ermittlung der durch Venturia inaequalis befallenen Blattfläche gegenüber der infizierten, nicht behandelten Kontrolle. Die Resultate sind in Tabelle II zusammengefasst.

| Substanz | Konzentration (in mg/l Spritzbrühe) | Wirkung (in %) | | |
|---|---|---|---|---|
| | | Erysiphe graminis | Puccinia coronata | Venturia inaequalis |
| 1-[3-(p-tert.-Butyl-phenyl)-2-methyl-propyl]-4,4-äthylendioxy-piperidin | 500 | | 100 | 20 |
| | 160 | 85 | 100 | |
| | 50 | 70 | 100 | |
| | 16 | 50 | 95 | |
| | 5 | 30 | 90 | |
| 1-[3-(p-tert.-Butyl-phenyl)-2-methyl-propyl]-4-piperidon | 500 | | 98 | 90 |
| | 160 | 100 | 0 | 10 |
| | 50 | 93 | 0 | 0 |
| | 16 | 80 | 0 | 0 |
| | 5 | 75 | 0 | 0 |
| 4-/3-[p-(2-Chlor-1,1-dimethyl-äthyl)-phenyl]-2-methyl-propyl/-2,6-dimethyl-morpholin | 500 | 100 | | 95 |
| | 160 | 100 | 100 | 95 |
| | 50 | 90 | 20 | 93 |
| | 16 | 75 | 0 | 67 |
| | 5 | 60 | 0 | 13 |
| α-(p-tert.-Butyl-phenyl)-β-methyl-1-piperidin-propanol | 500 | 98 | 100 | 100 |
| | 160 | 98 | 100 | 93 |
| | 50 | 87 | 80 | 43 |
| | 16 | 80 | 30 | 20 |
| | 5 | 75 | 10 | 10 |
| 1-[3-(p-tert.-Amyl-phenyl)-2,3-dimethyl-2-propenyl]-4,4-äthylendioxy-piperidin | 500 | | 100 | 95 |
| | 160 | 98 | 100 | 10 |
| | 50 | 90 | 100 | 10 |
| | 16 | 90 | 98 | 0 |
| | 5 | 75 | 20 | 0 |

| Substanz | Konzentration (in mg/l Spritzbrühe) | Wirkung (in %) | | |
|---|---|---|---|---|
| | | Erysiphe graminis | Puccinia coronata | Venturia inaequalis |
| 1-[3-(p-tert.-Amyl-phenyl)-2-methyl-propyl]-4,4-äthylendioxy-piperidin | 500<br>160<br>50<br>16<br>5 | 100<br>100<br>90<br>85<br>75 | 100<br>100<br>90<br>30<br>10 | 98<br>97<br>63<br>40<br>0 |
| 1-[3-(p-tert.-Amyl-phenyl)-2-methyl-propyl]-4-piperidon | 500<br>160<br>50<br>16<br>5 | 100<br>100<br>95<br>80<br>20 | 100<br>98<br>90<br>10<br>0 | 20 |
| 1-[3-(p-tert.-Amyl-phenyl)-2,3-dimethyl-2-propenyl]-4-piperidon | 500<br>160<br>50<br>16<br>5 | <br>98<br>90<br>85<br>60 | 100<br>100<br>100<br>95<br>90 | <br>98<br>83<br>37<br>0 |
| 1-[3-(p-tert.-Butyl-phenyl)-2,3-dimethyl-2-propenyl]-4,4-äthylendioxy-piperidin | 500<br>160<br>50<br>16<br>5 | 100<br>100<br>98<br>98<br>80 | 100<br>100<br>100<br>100<br>98 | 83<br>37<br>20<br>0<br>0 |
| 1-[3-(p-tert.-Butyl-phenyl)-2,3-dimethyl-2-propenyl]-4-piperidon | 500<br>160<br>50<br>16<br>5 | 100<br>100<br>100<br>60<br>0 | 100<br>100<br>100<br>25<br>0 | 83<br>37<br>20<br>10<br>0 |

| Substanz | Konzentration (in mg/l Spritzbrühe) | Wirkung (in %) | | |
|---|---|---|---|---|
| | | Erysiphe graminis | Puccinia coronata | Venturia inaequalis |
| 1-/3-[p-(2-Chlor-1,1-dimethyl-äthyl)-phenyl]-2-methyl-propyl/-piperidin | 500 | 100 | 100 | 95 |
| | 160 | 97 | 100 | 90 |
| | 50 | 80 | 85 | 30 |
| | 16 | 70 | 65 | 20 |
| | 5 | 45 | 30 | 0 |
| 1-/3-[p-(2-Chlor-1,1-dimethyl-äthyl)-phenyl]-2-methyl-propyl/-3,5-dimethyl-piperidin | 500 | 100 | 100 | 99 |
| | 160 | 100 | 100 | 87 |
| | 50 | 100 | 100 | 77 |
| | 16 | 92 | 98 | 33 |
| | 5 | 85 | 40 | 20 |

Die Verwendung der erfindungsgemässen Mittel kann nach den im Pflanzenschutz üblichen Applikationsmethoden erfolgen. Ein Gemisch kann in geeigneten Lösungsmitteln gelöst, in Emulsionen oder Dispersionen übergeführt, oder auf geeignete Trägerstoffe aufgebracht werden. Ausser den inerten Verteilungsmitteln kann man der Mischung auch noch konventionelle insektizide, akarizide, bakterizide und/ oder andere fungizide Verbindungen zusetzen, so dass man Pflanzenschutzmittel mit einer grossen Wirkungsbreite erhält. Genannt seien beispielsweise: O,O-Dimethyl-S-(1,2-dicarbäthoxyäthyl)-dithiophosphat, O,O-Diäthyl-O-(p-nitrophenyl)-thiophosphat, γ-Hexachlorcyclohexan, 2,2-bis-(p-Aethylphenyl)-1,1-dichloräthan, p-Chlorbenzyl-p-chlorphenyl-sulfid, 2,2-bis-(p-Chlorphenyl)-1,1,1-trichloräthanol, Zink-äthylen-bisdithiocarbamat, N-Trichlormethylthiotetrahydrophthalimid, Schwefel usw.

Zur Herstellung von pulverförmigen Präparaten kommen verschiedene inerte pulverförmige Trägerstoffe in Frage, wie z.B. Kaolin, Bentonit, Talkum, Schlämmkreide, Magnesiumcarbonat oder Kieselgur. Die aktiven Komponenten werden mit solchen Trägerstoffen vermischt, z.B. durch Zusammenmahlen; oder man imprägniert den inerten Trägerstoff mit einer Lösung der aktiven Komponenten und entfernt dann das Lösungsmittel durch Abdunsten, Erhitzen oder durch Absaugen unter vermindertem Druck. Solche pulverförmige Präparate können als Stäubemittel mit Hilfe der üblichen Verstäubergeräte auf die zu schützenden Pflanzen aufgebracht werden. Durch Zusatz von Netz- und/oder Dispergiermitteln kann man solche pulverförmige Präparate mit Wasser leicht benetzbar machen, so dass sie in Form von wässerigen Suspensionen als Spritzmittel anwendbar sind.

Zur Herstellung emulgierbarer Konzentrate können die aktiven Stoffe beispielsweise mit einem Emulgiermittel gemischt oder auch in einem inerten Lösungsmittel gelöst und mit einem Emulgator gemischt werden. Durch Verdünnen solcher Konzentrate mit Wasser erhält man gebrauchsfertige Emulsionen.

Die erfindungsgemässen Wirkstoffe der Formel I sind teilweise auf Grund ihrer fungistatischen und fungiziden Wirkung auch geeignet zur Bekämpfung von Infektionen, die durch Pilze und Hefen hervorgerufen werden, beispielsweise der Genera Candida, Trichophyten oder Histoplasma. Sie sind insbesondere wirksam gegen Candida-Arten wie Candida albicans und eignen sich vorzugsweise zur lokalen Therapie oberflächlicher Infektionen der Haut und der Schleimhäute, insbesondere des Genitaltraktes, beispielsweise Vaginitis, speziell verursacht durch Candida. Die Applikationsform der Wahl ist die lokale, so dass die Wirkstoffe als therapeutisch aktive Präparate in Form von Salben, Zäpfchen, Suppositorien, Ovula oder anderen geeigneten Formen zur Anwendung kommen können.

Die Herstellung der pharmazeutischen Präparate kann in an sich bekannter Weise durch Vermischen der Wirkstoffe mit üblichen organischen oder anorganischen inerten Trägermaterialien und/oder Hilfsstoffe, wie Wasser, Gelatine, Milchzucker, Stärke, Magnesiumstearat, Talk, pflanzliche Oele, Polyalkylenglykole, Vaseline, Konservierungs-, Stabilisierungs-, Netz- oder Emulgiermittel, Salze zur Veränderung des osmotischen Druckes oder Puffer, erfolgen.

Die Dosierung erfolgt nach individuellen Erfordernissen, jedoch dürfte eine Applikation von täglich 1-2 Tabletten die 100 mg Wirkstoff enthalten während weniger Tage eine bevorzugte Dosierung darstellen. Die Salben enthalten zweckmässigerweise 0,3-5%, vorzugsweise 0,5-2%, besonders bevorzugt 0,5-1% an Wirkstoff. Die nachstehenden Versuchsberichte und die in Tabelle II angegebenen Resultate geben dem Fachmann ebenfalls die notwendige Information für die Dosierung der Wirkstoffe.

a) Test: Candida albicans in vitro

Methode: Eine standardisierte Suspension der Hefeform von Candida albicans Stamm H 29 (ca. 300 Zellen/5 ml, das fünfzigfache der zum Angehen der Kultur notwendigen geringsten Keimzahl) wird gleichzeitig mit geeigneten Präparatlösungen in verflüssigten und auf 50°C abgekühlten Agarnährboden nach Rowley und Huber eingegossen. Lösung der Präparate in Wasser oder Polyäthylenglykol (Carbowax 400). Präparate, die weder in Wasser noch in Polyäthylenglykol löslich sind, werden fein suspendiert. Endkonzentrationen der Präparate im Nährboden 100, 10 und 1 mcg/ml. Endkonzentration von Polyäthylenglykol 5%. 7 Tage Bebrütung bei 37°C.

Auswertung: Ablesung des Pilzwachstums mit blossem Auge.

Resultate: Angabe der minimalen Präparatkonzentration in mcg/ml, die das Wachstum des Pilzes vollständig verhindert (MHC). Die Resultate sind für einige Beispiele in der nachstehenden Tabelle II zusammengefasst.

b) Test: Trichophyton mentagrophytes in vitro

Methode: Eine standardisierte Suspension der Hefeform von Konidien (Sporen) von Trichophyten mentagrophytes Stamm 109 (ca. das Fünfzigfache der zum Angehen der Kultur notwendigen geringsten Keimzahl) wird gleichzeitig mit geeigneten Präparatlösungen in verflüssigten und auf 50°C abgekühlten Agarnährboden nach Rowley und Huber eingegossen. Lösung der Präparate in Wasser oder Polyäthylenglykol (Carbowax 400). Präparate, die weder in Wasser noch in Polyäthylenglykol löslich sind, werden fein suspendiert. Endkonzentrationen der Präparate im Nährboden 100, 10, 1, 0,1 und 0,01 mcg/ml. Endkonzentration von Polyäthylenglykol 5%. 7 Tage Bebrütung bei 37°C.

Auswertung: Ablesung des Pilzwachstums mit blossem Auge.

Resultate: Angabe der minimalen Präparatkonzentration in mcg/ml, die das Wachstum des Pilzes vollständig verhindert (MHC). Die Resultate sind für einige Beispiele in der nachstehenden Tabelle II zusammengefasst.

c) Test: Histoplasma capsulatum in vitro

Methode: Eine standardisierte Suspension der Hefeform von Histoplasma capsulatum Stamm Hist 2 (ca. das Fünfzigfache der zum Angehen der Kultur notwendigen geringsten Keimzahl) wird gleichzeitig mit geeigneten Präparatlösungen in verflüssigten und auf 50°C abgekühlten Agarnährboden nach Rowley und Huber eingegossen. Lösung der Präparate in Wasser oder Polyäthylenglykol (Carbowax 400). Präparate, die weder in Wasser noch in Polyäthylenglykol löslich sind, werden fein suspendiert. Endkonzentration der Präparate im Nährboden 100, 10, 1, 0,1 und 0,01 mcg/ml. Endkonzentration von Polyäthylenglykol 5%. 12 Tage Bebrütung bei 28°C.

Auswertung: Ablesung des Pilzwachstums mit blossem Auge.

Resultate: Angabe der minimalen Präparatkonzentration in mcg/ml, die das Wachstum des Pilzes vollständig verhindert (MHC). Die Resultate sind für einige Beispiele in der nachstehenden Tabelle II zusammengefasst.

Tabelle II

| Substanz | MHC (µg/ml) | | |
|---|---|---|---|
| | Candida albicans | Trichophyton mentagr. | Histoplasma capsulatum |
| 1-[3-(p-tert.-Amyl-phenyl)-2-methyl-propyl]-4-piperidon | 10 | 10 | 10 |
| 1-[3-(p-tert.-Amyl-phenyl)-2,3-dimethyl-2-propenyl]-4-piperidon | 10 | 10 | 10 |

MHC = minimale Hemmkonzentration.

Die angegebenen Werte stellen grösstenteils Maximalwerte dar, d.h. die minimale Hemmkonzentration kann tiefer liegen.

Die nachstehenden Beispiele illustrieren die Erfindung. Alle Temperaturen sind in °C angegeben.

I. Herstellung des in den biologischen Versuchen verwendeten Spritzpulvers und weiterer Formulierungen:

1. Spritzpulver für alle Verbindungen der Formel I

Beispiel 1

| | | w/w % * |
|---|---|---|
| | Wirkstoff | 25,0 |
| a) | Silcasil S (BAYER) | 25,0 |
| b) | Tylose MH 1000 (HOECHST) | 1,0 |
| | Na-oleat | 2,0 |
| c) | Imbentin N-52 (KOLB) | 3,0 |
| d) | Ekapersol N (UGINE-KUHLMANN) | 10,0 |
| | Kaolin B 24 | 34,0 |
| | | 100,0 |

a) feinteilige hydratisierte Kieselsäure
b) Methylhydroxyäthylcellulose
c) Nonylphenol-Aethylenoxid-Addukt
d) Na-Salz der Dinaphthylmethandisulfosäure

* Gewichtsprozent

Die festen Wirkstoffe werden mit Silcasil S vermischt bzw. flüssige Wirkstoffe auf Silcasil S aufgezogen. Die übrigen Zuschlagsstoffe werden zugegeben und das Ganze in einer geeigneten Vorrichtung homogen vermischt. Das entstandene Pulver wird nun in einem geeigneten Mahlaggregat (z.B. Stiftenmühle, Hammermühle, Kugelmühle, Luftstrahlmühle etc.) feingemahlen und hernach nochmals gemischt.

2. Saatbeizmittel für alle Verbindungen der Formel I

Beispiel 2

| | % w/w |
|---|---|
| Wirkstoff | 20,0 |
| Ca-silikat | 20,0 |
| Rotes Eisenoxidpigment | 8,0 |
| Roter Xanthen-Farbstoff (Color Index: Solvent Red 49) | 0,5 |
| Stärkehydrolysat-Pulver (Dextrin) | 2,0 |
| Sulfitzellstoffablauge-Pulver | 3,2 |
| Na-Butylnaphthylsulfonat | 2,0 |
| Kaolin b 24 | 44,3 |
| | 100,0 |

Der feste Wirkstoff wird mit Calciumsilikat vermischt bzw. flüssiger Wirkstoff auf Calciumsilikat aufgezogen. Die übrigen Zuschlagsstoffe werden zugegeben und das Ganze gemischt und gemahlen (vgl. Beispiel 1). Das vorliegende rote Pulver kann tel quel als Trockenbeizmittel oder mit Wasser verdünnt als Nassbeizmittel für Saatgut verwendet werden.

3. Emulgierbare Konzentrate für öllösliche Verbindung der Formel I

Beispiel 3

| | | g/l |
|---|---|---|
| Wirkstoff (z.B. 1-[3-(p-tert.-Amyl-phenyl)-2-methyl-propyl]-4,4-äthylendioxy-piperidin usw.) | | 500 |
| Rizinusöl-Aethylenoxid-Addukt | | 100 |
| Ca-Salz der Dodecylbenzolsulfonsäure | | 50 |
| N-Methyl-2-pyrrolidon | | 100 |
| Aromatisches Lösungsmittel (Gemisch von $C_{10}$-Alkylbenzolen) | ad | 1000 ml |

Der Wirkstoff wird in einem Anteil des aromatischen Lösungsmittels gelöst, hernach die übrigen Zuschlagsstoffe zugesetzt, gelöst und mit dem Lösungsmittel zur Marke gestellt. Das vorliegende Produkt wird zur Herstellung der gebrauchsfertigen Spritzbrühe in Wasser gegeben, wobei eine für Stunden stabile Emulsion (O/W) entsteht.

4. Formulierungen für Verbindungen der Formel I mit einem protonisierbaren Stickstoff

Dieser Formulierungstyp enthält Salze und Molekül- und Additionsprodukte der erfindungsgemässen Substanzen, z.B.

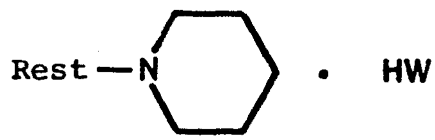

wobei HW eine Säure oder ein Säuregemisch bedeutet, welche bzw. welches vorzugsweise einen pK-Wert von < 5,0 aufweist.

In Frage kommen vorzugsweise organische Säuren, welche Salze bilden, die in Wasser, in Gemischen von Wasser mit wasserlöslichen Lösungsmitteln und in nicht polaren Lösungsmitteln löslich sind.

Die Herstellung der Salze erfolgt vorzugsweise in situ bei der Formulierung der erfindungsgemässen Wirkstoffe durch Zugabe der stöchiometrischen Mengen der Säuren der Formel HW in Anwesenheit von Wasser und/oder organischen Lösungsmitteln oder festen Trägerstoffen bei üblichen Temperaturen.

**Beispiel 4**

| | | g/l |
|---|---|---|
| Wirkstoff (z.B. 1-[3-(p-tert.-Amyl-phenyl)-2-methyl-propyl]-4,4-äthylendioxy-piperidin | | 250 |
| Essigsäure (100%) (pK: 4,75) | | 35 |
| Milchsäure (90%) (pK: 3,08) | | 26 |
| Isopropanol | | 300 |
| Wasser, entionisiert | ad | 1000 ml |

Isopropanol wird vorgelegt und der Wirkstoff darin gelöst. Unter Rühren werden die Milch- und die Essigsäure zugegeben, wobei eine relativ starke Wärmetönung entsteht. Mit Wasser wird zur Marke aufgefüllt. Die entstandene klare, praktisch farblose Lösung (ein wasserlösliches Konzentrat) kann mit Wasser zu einer gebrauchsfertigen Spritzbrühe verdünnt werden.

### Beispiel 5

| | | g/l |
|---|---|---|
| Wirkstoff (z.B. 1-[3-(p-tert.-Amyl-phenyl)-2-methyl-propyl]-4,4-äthylendioxy-piperidin | | 250 |
| Methansulfonsäure | | 70 |
| Wasser, entionisiert | ad | 1000 ml |

Ein Teil des Wassers wird vorgelegt und unter Rühren tropfenweise die Methansulfonsäure zugegeben, wobei eine sehr starke Wärmetönung entsteht. Nach dem Abkühlen auf Raumtemperatur wird mit Wasser zur Marke aufgefüllt. Die entstandene klare, schwach gelbliche Lösung (ein wasserlösliches Konzentrat) kann mit Wasser zu einer gebrauchsfertigen Spritzbrühe verdünnt werden.

### Beispiel 6

| | | g/l |
|---|---|---|
| Wirkstoff (z.B. 1-[3-(p-tert.-Amyl-phenyl)-2-methyl-propyl]-4,4-äthylendioxy-piperidin | | 250 |
| Bis-(2-äthylhexyl)-phosphorsäure | | 116 |
| Tensiofix BS (Emulgator) | | 100 (*) |
| Aromatisches Lösungsmittel (Gemisch von $C_{10}$-Alkylbenzolen) | ad | 1000 ml |

(*) Produkt der Firma TENSIA, Liège, Belgien: Gemisch aus Nonylphenol-Aethylenoxid-Addukten, Dodecylbenzolsulfonsäure-Calcium-Salz und Lösungsmittel

Der Wirkstoff wird in einem Teil des benötigten aromatischen Lösungsmittels gelöst und hierauf die Bis-(2-äthyl-

hexyl)-phosphorsäure tropfenweise eingerührt, wobei eine Wärmetönung entsteht. Die noch warme Mischung wird mit dem Emulgator versetzt und nach dem Abkühlen auf Raumteperatur mit dem aromatischen Lösungsmittel zur Marke gestellt. Zur Herstellung der gebrauchsfertigen Spritzbrühe wird das vorliegende Produkt (ein emulgierbares Konzentrat) in Wasser eingerührt, wobei eine Emulsion (O/W) entsteht.

Beispiel 7

| | | g/l |
|---|---|---|
| Wirkstoff (z.B. 1-[3-(p-tert.-Amyl-phenyl)-2-methyl-propyl]-4,4-äthylendioxy-piperidin | | 250 |
| Phosphorsäuremono- und -diester aus Nonylphenolpolyglykoläther | | 320 |
| Dimethylformamid | | 200 |
| 1,1,1-Trichloräthan | ad | 1000 ml |

Der Wirkstoff wird in Dimethylformamid gelöst und hierauf der Phosphorsäureester tropfenweise eingerührt, wobei eine merkliche Wärmetönung entsteht. Nach dem Abkühlen wird mit 1,1,1-Trichloräthan zur Marke gefüllt. Zur Herstellung der fertigen Spritzbrühe wird das vorliegende Produkt (ein emulgierbares Konzentrat) in Wasser eingerührt, wobei eine für Stunden stabile Emulsion (O/W) entsteht.

Ein typisches Merkmal dieser Formulierung ist die Verwendung einer tensioaktiven Säure, welche den Zusatz eines Emulgators überflüssig macht.

Beispiel 8

| | w/w % |
|---|---|
| Wirkstoff (z.B. 1-[3-p-tert.-Amyl-phenyl)-2-methyl-propyl]-4,4-äthylendioxy-piperidin | 25,0 |
| Sulfaminsäure | 7,0 |
| Silcasil S | 25,0 |
| Mischung aus 85% Na-Dioctylsulfo-succinat und 15% Na-benzoat | 1,0 (*) |
| Diammoniumhydrogenphosphat | 40,0 |

(*) Produkt (Aerosol OT-B) der American Cynamid; US-Pat. No. 2.441.341

Der Wirkstoff wird mit Silcasil S vermischt, wobei ein trockenes Pulver entsteht. Hierauf werden die restlichen Zuschlagsstoffe beigemischt und das Ganze in einem geeigneten Mahlaggregat (vgl. Beispiel 1) feingemahlen. Zur Herstellung der fertigen Spritzbrühe wird das vorliegende Produkt (ein wasserlösliches Pulver) mit Wasser verdünnt.

II. Herstellung der Wirkstoffe:

Beispiel 9

9,4 g 3-[p-(2-Chlor-1,1-dimethyl-äthyl)-phenyl]-2-methyl-propionaldehyd und 3,78 g Piperidin werden in 50 ml Toluol mit einem Wasserabscheider unter Stickstoffbegasung 16 Stunden bis zur Beendigung der Wasserabspaltung am Rückfluss erhitzt und danach am Rollverdampfer eingeengt. Der Rückstand wird unter Stickstoffspülung mit 50 ml Aethanol zu 0,6 g 5% Palladium auf Kohle gespült und bis zur Beendigung der Wasserstoffaufnahme hydriert. Die Lösung wird vom Katalysator abgetrennt, eingeengt und destilliert. Es wird reines 1-/3-[p-(2-Chlor-1,1-dimethyl-äthyl)-phenyl]-2-methyl-propyl/-piperidin vom Siedepunkt 140$^{o}$/0,04 Torr erhalten.

In analoger Weise erhält man ausgehend von:

- 3-[p-(2-Chlor-1,1-dimethyl-äthyl)-phenyl]-2-methyl-propionaldehyd mit 3,5-Dimethyl-piperidin durch Wasserabspaltung und anschliessender Hydrierung das 1-/3-[p-(2-Chlor-1,1-dimethyl-äthyl)-phenyl]-2-methyl-propyl/-3,5-dimethyl-piperidin, Sdp. 156°/0,04 Torr,

- 3-[p-(2-Chlor-1,1-dimethyl-äthyl)-phenyl]-2-methyl-propionaldehyd mit 2,6-Dimethyl-morpholin durch Wasserabspaltung und anschliessender Hydrierung das 4-/3-[p-(2-Chlor-1,1-dimethyl-äthyl)-phenyl]-2-methyl-propyl/-2,6-dimethyl-morpholin, Sdp. 155°/0,035 Torr,

- 3-[p-(2-Chlor-1,1-dimethyl-äthyl)-phenyl]-2-methyl-propionaldehyd mit 4,4-Aethylendioxy-piperidin durch Wasserabspaltung und anschliessender Hydrierung das 1-/3-[p-(2-Chlor-1,1-dimethyl-äthyl)-phenyl]-2-methyl-propyl/-4,4-äthylendioxy-piperidin,

- 3-(p-tert.-Butyl-phenyl)-2-methyl-propionaldehyd mit 4,4-Aethylendioxy-piperidin durch Wasserabspaltung und anschliessender Hydrierung das 1-[3-(p-tert.-Butyl-phenyl)-2-methyl-propyl]-4,4-äthylendioxy-piperidin, Sdp. 146-153°/0,024 Torr,

- 3-(p-tert.-Butyl-phenyl)-2-methyl-propionaldehyd mit 4,4-äthylendioxy-3-methyl-piperidin durch Wasserabspaltung und anschliessender Hydrierung das 1-[3-(p-tert.-Butyl-phenyl)-2-methyl-propyl]-4,4-äthylendioxy-3-methyl-piperidin,

- 3-(p-tert.-Butyl-phenyl)-2-methyl-propionaldehyd mit 4,4-Dimethoxy-piperidin durch Wasserabspaltung und anschliessender Hydrierung das 1-[3-(p-tert.-Butyl-phenyl)-2-methyl-propyl]-4,4-dimethoxy-piperidin,

- 3-(p-tert.-Butyl-phenyl)-2-methyl-propionaldehyd mit 3-Methyl-4-piperidon durch Wasserabspaltung und anschliessender Hydrierung das 1-[3-(p-tert.-Butyl-phenyl)-2-methyl-propyl]-3-methyl-4-piperidon,

- 3-(p-tert.-Butyl-phenyl)-2-methyl-propionaldehyd mit 3,5-Dimethyl-4-piperidon durch Wasserabspaltung und anschliessender Hydrierung das 1-[3-(p-tert.-Butyl-phenyl)-2-methyl-propyl]-3,5-dimethyl-4-piperidon,

- 3-(p-tert.-Amyl-phenyl)-2-methyl-propionaldehyd mit 4,4-Aethylendioxy-piperidin durch Wasserabspaltung und anschliessender Hydrierung das 1-[3-(p-tert.-Amyl-phenyl)-2-methyl-propyl]-4,4-äthylendioxy-piperidin, Sdp. 175°/0,055 Torr,

- 3-(p-tert.-Amyl-phenyl)-2-methyl-propionaldehyd mit 4,4-Aethylendioxy-3-methyl-piperidin durch Wasserabspaltung und anschliessender Hydrierung das 1-[3-(p-tert.-Amyl-phenyl)-2-methyl-propyl]-4,4-äthylendioxy-3-methyl-piperidin,

- 3-(p-tert.-Amyl-phenyl)-2-methyl-propionaldehyd mit 4,4-Dimethoxy-piperidin durch Wasserabspaltung und anschliessender Hydrierung das 1-[3-(p-tert.-Amyl-phenyl)-2-methyl-propyl]-4,4-dimethoxy-piperidin,

- 3-(p-tert.-Amyl-phenyl)-2-methyl-propionaldehyd mit 3-Methyl-4-piperidon durch Wasserabspaltung und anschliessender Hydrierung das 1-[3-(p-tert.-Amyl-phenyl)-2-methyl-propyl]-3-methyl-4-piperidon,

- 3-(p-tert.-Amyl-phenyl)-2-methyl-propionaldehyd mit 3,5-Dimethyl-4-piperidon durch Wasserabspaltung und anschliessender Hydrierung das 1-[3-(p-tert.-Amyl-phenyl)-2-methyl-propyl]-3,5-dimethyl-4-piperidon,

- 3-[p-(1-Cyclohexyl-1-methyl)-phenyl]-2-methyl-propionaldehyd mit 4,4-Aethylendioxy-piperidin durch Wasserabspaltung und anschliessender Hydrierung das 1-/3-[p-(1-Cyclohexyl-1-methyl)-phenyl]-2-methyl-propyl/-4,4-äthylendioxy-piperidin.

## Beispiel 10

Zu einer Lösung von 48,6 g 4,4-Aethylendioxy-piperidin in 150 ml Aether werden 35 g Triäthylamin und anschliessend eine Lösung von 97 g 3-(p-tert.-Amyl-phenyl)-2,3-dimethyl-allylbromid in 110 ml Aether zugetropft und 16 Stunden rückflussiert. Das Reaktionsgemisch wird auf Wasser gegossen, die Aetherphase abgetrennt und die wässrige Phase mit Aether nachextrahiert. Die vereinigten Aetherphasen werden mit Natriumbicarbonatlösung und Wasser gewaschen, über Natriumsulfat getrocknet und eingedampft. Durch Destillation des Rückstandes wird reines 1-[3-(p-tert.-Amyl-phenyl)-2,3-dimethyl-2-propenyl]-4,4-äthylendioxy-piperidin, Sdp. 163°/0,04 Torr erhalten.

In analoger Weise erhält man ausgehend von:

- 3-(p-tert.-Butyl-phenyl)-2,3-dimethyl-allylbromid und 4,4-Aethylendioxy-piperidin das 1-[3-(p-tert.-Butyl-phenyl)-2,3-dimethyl-2-propenyl]-4,4-äthylendioxy-piperidin, Sdp. 146°/0,04 Torr,

- 3-[p-(1-Cyclohexyl-1-methyl)-phenyl]-2,3-dimethyl-allylbromid und 4,4-Aethylendioxy-piperidin das 1-/3-[p-(1-Cyclohexyl-1-methyl)-phenyl]-2,3-dimethyl-2-propenyl/-4,4-äthylendioxy-piperidin,

- 3-[p-(2-Chlor-1,1-dimethyl-äthyl)-phenyl]-2,3-dimethyl-allylbromid und Piperidin das 1-/3-[p-(2-Chlor-1,1-dimethyl-äthyl)-phenyl]-2,3-dimethyl-2-propenyl/-piperidin,

- 3-[p-(2-Chlor-1,1-dimethyl-äthyl)-phenyl]-2,3-dimethyl-allylbromid und 3,5-Dimethyl-piperidin das

1-/3-[p-(2-Chlor-1,1-dimethyl-äthyl)-phenyl]-2,3-dimethyl-2-propenyl/-3,5-dimethyl-piperidin,

- 3-[p-(2-Chlor-1,1-dimethyl-äthyl)-phenyl]-2,3-dimethyl-allylbromid und 2,6-Dimethyl-morpholin das 4-/3-[p-(2-Chlor-1,1-dimethyl-äthyl)-phenyl]-2,3-dimethyl-2-propenyl/-2,6-dimethyl-morpholin,

- 3-[p-(2-Chlor-1,1-dimethyl-äthyl)-phenyl]-2,3-dimethyl-allylbromid und 4,4-Aethylendioxy-piperidin das 1-/3-[p-(2-Chlor-1,1-dimethyl-äthyl)-phenyl]-2,3-dimethyl-2-propenyl/-4,4-äthylendioxy-piperidin.

## Beispiel 11

Ein Gemisch von 25 g 1-[3-(p-tert.-Butyl-phenyl)-2-methyl-propyl]-4,4-äthylendioxy-piperidin in 115 ml Ameisensäure werden 6 Stunden bei 100° gerührt und anschliessend auf Eiswasser gegossen, mit konz. Natronlauge alkalisch gestellt und mit Aether extrahiert. Die vereinigten Aetherextrakte werden mit Wasser neutralgewaschen, über Natriumsulfat getrocknet und eingeengt. Durch Destillation erhält man reines 1-[3-(p-tert.-Butyl-phenyl)-2-methyl-propyl]-4-piperidon, Sdp. 135°/0,04 Torr.

In analoger Weise erhält man ausgehend von:

- 1-[3-(p-tert.-Butyl-phenyl)-2,3-dimethyl-2-propenyl]-4,4-äthylendioxy-piperidin das 1-[3-(p-tert.-Butyl-phenyl)-2,3-dimethyl-2-propenyl]-4-piperidon, Sdp. 150°/0,05 Torr,

- 1-[3-(p-tert.-Amyl-phenyl)-2-methyl-propyl]-4,4-äthylendioxy-piperidin das 1-[3-(p-tert.-Amyl-phenyl)-2-methyl-propyl]-4-piperidon, Sdp. 145°/0,05 Torr,

- 1-[3-(p-tert.-Amyl-phenyl)-2,3-dimethyl-2-propenyl]-4,4-äthylendioxy-piperidin das 1-[3-(p-tert.-Amyl-phenyl)-2,3-dimethyl-2-propenyl]-4-piperidon, Sdp. 135°/0,04 Torr,

- 1-/3-[p-(1-Cyclohexyl-1-methyl)-phenyl]-2-methyl-propyl/-4,4-äthylendioxy-piperidin das 1-/3-[p-(1-Cyclohexyl-1-methyl)-phenyl]-2-methyl-propyl/-4-piperidon,

- 1-/3-[p-(1-Cyclohexyl-1-methyl)-phenyl]-2,3-dimethyl-2-propenyl/-4,4-äthylendioxy-piperidin das 1-/3-[p-(1-Cyclohexyl-1-methyl)-phenyl]-2,3-dimethyl-2-propenyl/-4-piperidon,

- 1-/3-[p-(2-Chlor-1,1-dimethyl-äthyl)-phenyl]-2-methyl-propyl/-4,4-äthylendioxy-piperidin das 1-/3-[p-(2-Chlor-1,1-dimethyl-äthyl)-phenyl]-2-methyl-propyl/-4-piperidon,

- 1-/3-[p-(2-Chlor-1,1-dimethyl-äthyl)-phenyl]-2,3-dimethyl-2-propenyl/-4,4-äthylendioxy-piperidin das 1-/3-[p-(2-Chlor-1,1-dimethyl-äthyl)-phenyl]-2,3-dimethyl-2-propenyl/-4-piperidon.

## Beispiel 12

Zu einer Lösung von 75,6 g 4'-tert.-Butyl-2-methyl-3-piperidin-propiophenon in 930 ml Methanol-Wasser (9:1) wird während 20 Minuten bei 20-30° 14,9 g Natriumborhydrid in Portionen zugegeben und 2 Stunden bei Raumtemperatur nachgerührt. Die Reaktionslösung wird auf 1800 ml Wasser gegossen und mit Hexan extrahiert. Die vereinigten Hexanphasen werden über Natriumsulfat getrocknet, eingeengt und destilliert. Es wird reines α-(p-tert.-Butyl-phenyl)-β-methyl-1-piperidin-propanol vom Sdp. 166-172°/0,035 Torr erhalten.

Beispiel 13

Zu 12,6 g α-(p-tert.-Butyl-phenyl)-β-1-piperidin-propanol wird während 15 Minuten bei 65-70° 10,2 g Thionylchlorid zugetropft, 20 Minuten bei 65-70° und 1 Stunde bei 80-90° nachgerührt. Nach dem Abkühlen auf Raumtemperatur wird vorsichtig 400 ml gesättigte Natriumbicarbonatlösung zugegeben und das Produkt mit Dichlormethan extrahiert. Die vereinigten Dichlormethanextrakte werden über Natriumsulfat getrocknet, eingedampft, 50 ml Aether und 20 ml mit Chlorwasserstoff gesättigter Aether zugegeben, 15 Minuten bei Raumtemperatur stehen gelassen und abfiltriert. Die Kristalle werden mit 400 ml gesättigter Natriumbicarbonatlösung und 20 ml Aethanol verrührt und das freigesetzte Produkt mit Aether extrahiert. Die vereinigten Aetherauszüge werden mit Wasser gewaschen, über Natriumsulfat getrocknet und eingeengt. Der Rückstand wird über Kieselgel chromatographiert und das Eluat (Aether/Methanol 9:1) eingeengt. Durch Destillation wird reines 1-[3-(p-tert.-Butyl-phenyl)-3-chlor-2-methyl-propyl]-piperidin vom Siedepunkt 170°/0,05 Torr (Kugelrohrofen) erhalten.

Beispiel 14

23,4 g 3-(p-tert.-Butyl-phenyl)-3-methoxy-2-methyl-propionaldehyd, 9,4 g Piperidin, 1,2 g 5% Palladium auf Kohle und 200 ml Methanol werden unter Stickstoffspülung zusammengegeben und bei Raumtemperatur bis zur Aufnahme der theoretischen Wasserstoffmenge hydriert. Danach wird die Lösung vom Katalysator abgetrennt und eingedampft. Der Rückstand wird in 100 ml 15%iger Salzsäure aufgenommen und mit Aether gewaschen. Anschliessend wird die wässrig-salzsaure Lösung mit 15%iger Natronlauge alkalisch gestellt und das Produkt mit Aether extrahiert. Die vereinigten Aetherphasen werden mit Wasser gewaschen, über Natriumsulfat getrocknet und eingeengt. Durch Destillation wird reines 1-[3-(p-tert.-Butyl-phenyl)-3-methoxy-2-methyl-propyl]-

piperidin vom Siedepunkt 134-135°/0,05 Torr erhalten.

In den nachstehenden Beispielen wird die Herstellung der Ausgangsmaterialien beschrieben:

## Beispiel 15

Zu einer Mischung von 72,9 g Piperidinhydrochlorid und 27,0 g Paraformaldehyd in 180 ml absolutem Alkohol wird während 45 Minuten bei Rückflusstemperatur 114,0 g p-t-Butylpropiophenon zugetropft und 30 Minuten bei Rückflusstemperatur gerührt. Anschliessend wird nochmals 36,0 g Paraformaldehyd zugegeben und 16 Stunden bei Rückflusstemperatur gerührt. Nach dem Abkühlen auf Raumtemperatur wird dem Gemisch 300 ml 15%ige Salzsäure zugegeben und das unumgesetzte p-t-Butylpropiophenon mit Aether extrahiert. Die wässrig-salzsaure Lösung wird mit 600 ml 15%iger Natronlauge alkalisch gestellt und das Produkt mit Aether extrahiert. Die vereinigten Aetherphasen werden mit Wasser gewaschen, über Natriumsulfat getrocknet und eingedampft. Es werden 39,8 g 4'-tert.-Butyl-2-methyl-3-piperidin-propiophenon erhalten, $n_D^{20}$: 1,5250.

## Beispiel 16

Zu 90,0 g p-tert.-Butyl-benzaldehyd-dimethylacetal werden unter Stickstoffbegasung bei 0° 5,1 ml 10% Zinkchlorid in Eisessig zugegeben. Anschliessend wird während 60 Minuten bei 10-15° 37,1 g Aethyl-propenyl-äther zugetropft und 30 Minuten bei 15-20° nachgerührt. Zu dieser Lösung werden 450 ml Aceton und 45 ml 2N Schwefelsäure gegeben, 17 Stunden bei Raumtemperatur stehen gelassen, danach auf 900 ml Wasser gegossen und das Produkt mit Hexan extrahiert. Die vereinigten Hexanauszüge werden mit gesättigter Natriumbicarbonatlösung gewaschen, über Natriumsulfat getrocknet und eingeengt. Durch Destillation wird reiner 3-(p-tert.-Butyl-phenyl)-3-methoxy-2-methyl-propionaldehyd vom Siedepunkt 105-107°/0,06 Torr erhalten.

Zu einer Lösung von 1,4 g Kaliumhydroxid in 100 ml Methanol werden unter Stickstoffbegasung 108,5 g p-tert.-Butyl-benzaldehyd zugegeben und anschliessend bei 40° während 6 Stunden 39,2 g Propionaldehyd zugetropft. Anschliessend wird noch 1 Stunde bei 40° weitergerührt, 1,5 ml Essigsäure zugegeben und am Rotationsverdampfer eingeengt. Die ölige Suspension wird in Aether aufgenommen, mit Wasser neutralgewaschen, getrocknet und eingedampft. Durch Destillation wird reines 3-(p-tert.-Butyl-phenyl)-2-methyl-acrolein vom Siedepunkt 165°/11 Torr erhalten.

In analoger Weise erhält man ausgehend von:

- p-tert.-Amyl-benzaldehyd und Propionaldehyd das 3-(p-tert.-Amyl-phenyl)-2-methyl-acrolein, Sdp. 117-120°/0,035 Torr,

- p-(2-Chlor-1,1-dimethyl-äthyl)-benzaldehyd und Propionaldehyd das 3-[p-(2-Chlor-1,1-dimethyl-äthyl)-phenyl]-2-methyl-acrolein, Sdp. 136-137°/0,04 Torr,

- p-(2-Cyclohexyl-1,1-dimethyl-äthyl)-benzaldehyd und Propionaldehyd das 3-[p-(2-Cyclohexyl-1,1-dimethyl-äthyl)-phenyl]-2-methyl-acrolein, Sdp. 143-148°/0,04 Torr,

- p-(1-Propyl-1-methyl-pentyl)-benzaldehyd und Propionaldehyd das 3-[p-(1-Propyl-1-methyl-pentyl)-phenyl]-2-methyl-acrolein, Sdp. 136°/0,05 Torr,

- p-(1-Cyclohexyl-1-methyl)-benzaldehyd und Propionaldehyd das 3-[p-(1-Cyclohexyl-1-methyl)-phenyl]-2-methyl-acrolein, Sdp. 140-145°/0,05 Torr.

## Beispiel 17

Zu einem Gemisch von 300 g p-tert.-Butyl-benzaldehyd und 300 g Methyläthylketon tropft man während 1 Stunde bei 15-20° 300 g 32%ige Salzsäure und lässt 22 Stunden bei Raumtemperatur rühren. Anschliessend wird das Reaktionsgemisch in 200 ml Aether aufgenommen, mit Wasser und gesättigter Natriumbicarbonatlösung gewaschen, über Natriumsulfat getrocknet und eingeengt. Durch fraktionierte Destillation wird reines 4-(p-tert.-Butyl-phenyl)-3-methyl-3-buten-2-on, Sdp. 120°/0,03 Torr erhalten.

## Beispiel 18

404,5 g 3-(p-tert.-Butyl-phenyl)-2-methyl-acrolein werden in 2500 ml Methanol gelöst und unter Eiskühlung portionenweise mit 38 g Natriumborhydrid versetzt. Anschliessend wird 2,5 Stunden bei Raumtemperatur nachgerührt, auf 2500 ml eiskalte 2n Salzsäure gegossen und mit Hexan erschöpfend extrahiert. Die vereinigten Hexanextrakte werden mit Wasser neutral gewaschen, über Natriumsulfat getrocknet und eingedampft. Vakuumdestillation liefert reinen 3-(p-tert.-Butyl-phenyl)-2-methyl-allylalkohol, Sdp. 119°/0,005 Torr.

In analoger Weise erhält man ausgehend von:

- 4-(p-tert.-Butyl-phenyl)-3-methyl-3-buten-2-on den 3-(p-tert.-Butyl-phenyl)-1,2-dimethyl-allylalkohol, Sdp. 107°/0,005 Torr.

## Beispiel 19

73,2 g 3-(p-tert.-Butyl-phenyl)-2-methyl-allylalkohol und 8,6 ml Pyridin in 700 ml n-Pentan werden auf -5° abgekühlt. Bei dieser Temperatur werden unter Rühren während 2 Stunden 15,2 ml Phosphortribromid in 700 ml n-Pentan zugetropft und 3 Stunden bei Raumtemperatur nachgerührt. Das

Reaktionsgemisch wird auf 500 g Eis gegossen und 30 Minuten verrührt, die Pentanphase abgetrennt und die wässrige Phase mit n-Pentan nachextrahiert. Die vereinigten n-Pentanphasen werden mit gesättigter Natriumbicarbonatlösung und Wasser neutral gewaschen, über Natriumsulfat getrocknet und eingedampft. Das am Hochvakuum destillierte 3-(p-tert.-Butyl-phenyl)-2-methyl-allylbromid siedet bei 123°/0,01 Torr.

<u>Anmerkung</u>:

Substituierte Allylbromide der allgemeinen Formel IIa (vgl. Reaktionsschema A und B) sind thermisch instabil. Bei deren Destillation findet teilweise Zersetzung statt. Es ist deshalb vorteilhaft, die aus der Reaktion erhaltenen Allylbromide ohne weitere Reinigung für die nächste Stufe einzusetzen.

In analoger Weise erhält man ausgehend von:

- 3-(p-tert.-Butyl-phenyl)-1,2-dimethyl-allylalkohol das 3-(p-tert.-Butyl-phenyl)-1,2-dimethyl-allylbromid, $n_D^{20}$: 1,5654,

- 3-(p-tert.-Butyl-phenyl)-2,3-dimethyl-allylalkohol das 3-(p-tert.-Butyl-phenyl)-2,3-dimethyl-allylbromid, $n_D^{20}$: 1,5505,

- 3-(p-tert.-Butyl-phenyl)-1,2,3-trimethyl-allylalkohol das 3-(p-tert.-Butyl-phenyl)-1,2,3-trimethyl-allylbromid, NMR (60 Mc, $CDCl_3$): C$\underline{H}$-1 = 5,05 ppm (q) und

- 3-(p-tert.-Butyl-cyclohexyl)-2-methyl-allylalkohol das 3-(4-tert.-Butyl-cyclohexyl)-2-methyl-allylbromid, Sdp. 94-98°/0,05 Torr.

Beispiel 20

Eine Mischung von 20,2 g p-tert.-Butyl-cyclohexan-1-carboxaldehyd, 52 g (α-Carbäthoxy-äthyliden)-triphenyl-phosphoran und 3,6 g Benzoesäure in 120 ml Toluol wird 16 Stunden unter Stickstoffbegasung am Rückfluss erhitzt und das Toluol abgedampft. Der ölig-kristalline Rückstand wird in 600 ml Methanol-Wasser (4:1) gelöst und mit Hexan erschöpfend extrahiert. Die vereinigten Hexanextrakte werden mit Natriumbicarbonatlösung und Wasser gewaschen, über Natriumsulfat getrocknet und eingedampft. Durch Destillation wird reiner 3-(p-tert.-Butyl-cyclohexyl)-2-methyl-acrylsäure-äthylester, Sdp. 99°/0,03 Torr, erhalten.

Beispiel 21

Zu einer Lösung von 27,6 g Natrium in 1,1 Liter absolutem Aethanol werden 285,8 g Triäthyl-α-phosphoniumpropionat gegeben. Nach einer Rührdauer von 5 Minuten werden 176,3 g p-t-Butyl-acetophenon innert 15 Minuten zugetropft und 24 Stunden am Rückfluss gerührt. Danach wird die Lösung abgekühlt, mit 4,4 l Wasser verrührt und mit Chloroform extrahiert. Die vereinigten Chloroformextrakte werden mit Wasser gewaschen, über Natriumsulfat getrocknet und eingeengt. Durch Destillation wird reiner 3-(p-tert.-Butyl-phenyl)-2,3-dimethyl-acrylsäure-äthylester, Sdp. 99°/0,005 Torr, erhalten.

Beispiel 22

Eine Lösung von 270 ml Morpholin und 1000 ml absolutem Toluol wird in 30-40 Minuten, bei 0°, zu 740 ml einer 70%igen Natrium-dihydro-bis-(2-methoxyäthoxy)-aluminat-Lösung in Toluol und 1200 ml absolutem Toluol getropft. Obige Lösung wird innert 1 Stunde, bei 0°, zu 78,0 g 3-(p-tert.-Butyl-phenyl)-2,3-dimethyl-acrylsäure-äthylester in 340 ml absolutem Toluol getropft. Danach wird 3/4 Stunden bei 0° gerührt, auf 3 l Wasser gegossen und HCl zugegeben,

bis die Emulsion beseitigt ist. Die Toluollösung wird abgetrennt, mit Wasser und Natriumbicarbonatlösung gewaschen, über Natriumsulfat getrocknet und eingeengt. Durch Destillation wird reiner p-tert.-Butyl-α,β-dimethyl-zimtaldehyd, Sdp. 122-128°/0,005 Torr, erhalten.

Beispiel 23

Aus 10,7 g Magnesium in 30 ml absolutem Aether und 68,8 g Methyljodid in 100 ml absolutem Aether wird in üblicher Weise eine Grignardlösung hergestellt. Zu dieser Lösung wird in 15-20 Minuten bei 20-25° 56,1 g p-tert.-Butyl-α,β-dimethyl-zimtaldehyd getropft. Nach dem Kühlen auf Raumtemperatur wird vorsichtig auf 200 g Eis gegossen und 150 g technisches Ammoniumchlorid in 500 ml Wasser zugegeben. Die organische Phase wird abgetrennt, mit Wasser und Natriumbicarbonatlösung gewaschen, über Natriumsulfat getrocknet und eingeengt. Durch Destillation wird einer 3-(p-tert.-Butyl-phenyl)-1,2,3-trimethyl-allylalkohol, Sdp. 143-148°/0,001 Torr, erhalten.

Beispiel 24

Zu einer Lösung von 25,3 g 3-(p-tert.-Butyl-cyclohexyl)-2-methyl-acrylsäure-äthylester in 130 ml abs. Toluol wird während 90 Minuten bei 25-30° 46 g einer 70%igen Natrium-dihydro-bis-(2-methoxyäthoxy)-aluminat-Lösung in Toluol zugetropft und anschliessend 2 Stunden bei 40° erwärmt. Dann wird auf -10° abgekühlt, mit 130 ml 2n Natronlauge tropfenweise versetzt, die Toluolphase abgetrennt und die wässrig-alkalische Phase zweimal mit 200 ml Toluol nachextrahiert. Die vereinigten Toluolphasen werden mit Wasser neutral gewaschen, über Natriumsulfat getrocknet und eingedampft. Durch Destillation wird reiner 3-(p-tert.-Butyl-cyclohexyl)-2-methyl-allylalkohol, Sdp. 112-114°/0,08 Torr, erhalten.

In analoger Weise erhält man ausgehend von:

- 3-(p-tert.-Butyl-phenyl)-2,3-dimethyl-acrylsäure-äthylester den 3-(p-tert.-Butyl-phenyl)-2,3-dimethyl-allylalkohol, Sdp. 107-110°/0,005 Torr.

## Beispiel 25

25,6 g 3-[p-(2-Chlor-1,1-dimethyl-äthyl)-phenyl]-2-methyl-acrolein, 1,3 g 5% Palladium auf Kohle und 0,1 g Calciumhydroxid werden unter Stickstoffspülung vorgelegt und eine Lösung von 1,5 ml Wasser in 65 ml Methanol zugegeben. Bei Raumtemperatur wird bis zur Absättigung der α,β-ständigen Doppelbindung hydriert, vom Katalysator abfiltriert, eingeengt und destilliert. Es wird reiner 3-[p-(2-Chlor-1,1-dimethyl-äthyl)-phenyl]-2-methyl-propionaldehyd vom Siedepunkt 110-113°/0,045 Torr erhalten.

In analoger Weise erhält man ausgehend von:

- 3-(p-tert.-Butyl-phenyl)-2-methyl-acrolein den 3-(p-tert.-Butyl-phenyl)-2-methyl-propionaldehyd, Sdp. 150°/10 Torr,

- 3-(p-tert.-Amyl-phenyl)-2-methyl-acrolein den 3-(p-tert.-Amyl-phenyl)-2-methyl-propionaldehyd, Sdp. 109-111°/0,06 Torr,

- 3-[p-(2-Cyclohexyl-1,1-dimethyl-äthyl)-phenyl]-2-methyl-acrolein den 3-[p-(2-Cyclohexyl-1,1-dimethyl-äthyl)-phenyl]-2-methyl-propionaldehyd, Sdp. 141-143°/0,045 Torr,

- 3-[p-(1-Propyl-1-methyl-pentyl)-phenyl]-2-methyl-acrolein den 3-[p-(1-Propyl-1-methyl-pentyl)-phenyl]-2-methyl-propionaldehyd, Sdp. 129-134°/0,05 Torr,

- 3-[p-(1-Cyclohexyl-1-methyl)-phenyl]-2-methyl-acrolein den 3-[p-(1-Cyclohexyl-1-methyl)-phenyl]-2-methyl-propionaldehyd, Sdp. 136-141°/0,05 Torr.

Beispiel 26

65 g 3-(p-tert.-Butyl-phenyl)-1,2-dimethyl-allylalkohol werden in 650 ml Alkohol gelöst, unter Stickstoffbegasung mit 6 g 5% Palladium auf Kohle versetzt und bis zur Beendigung der Wasserstoffaufnahme hydriert. Anschliessend wird vom Katalysator filtriert und der Alkohol abgedampft. Durch Destillation wird reines 3-(p-tert.-Butyl-phenyl)-1,2-dimethyl-propanol, Sdp. 110°/0,03 Torr erhalten.

In analoger Weise erhält man ausgehend von:

- 3-(p-tert.-Butyl-phenyl)-2-methyl-allylalkohol das 3-(p-tert.-Butyl-phenyl)-2-methyl-propanol, Sdp. 148-150°/10 Torr,

- 3-(p-tert.-Butyl-phenyl)-2,3-dimethyl-allylalkohol das 3-(p-tert.-Butyl-phenyl)-2,3-dimethyl-propanol und

- 3-(p-tert.-Butyl-phenyl)-1,2,3-trimethyl-allylalkohol das 3-(p-tert.-Butyl-phenyl)-1,2,3-trimethyl-propanol.

Beispiel 27

300,2 g 2-(p-tert.-Butyl-phenyl)-2-methyl-propanol werden während 2 Stunden bei 20-30° zu 218,6 g Phosphortribromid getropft und 16 Stunden stehen gelassen. Es wird anschliessend während 1,5 Stunden auf 55-60° erhitzt, auf ca. 10° abgekühlt und vorsichtig auf Eis gegossen, die wässrige Lösung mit Aether erschöpfend extrahiert, die vereinigten Aetherphasen mit gesättigter Natriumbicarbonatlösung und Wasser gewaschen, über Natriumsulfat getrocknet und eingedampft. Durch fraktionierte Destillation wird reines 2-(p-tert.-Butyl-phenyl)-2-methyl-propylbromid, Sdp. 104°/0,025 Torr erhalten.

In analoger Weise erhält man ausgehend von:

- 3-(p-tert.-Butyl-phenyl)-1,2-dimethyl-propanol das 3-(p-tert.-Butyl-phenyl)-1,2-dimethyl-propylbromid, Sdp. 112°/0,05 Torr,

- 3-(p-tert.-Butyl-phenyl)-2,3-dimethyl-propanol das 3-(p-tert.-Butyl-phenyl)-2,3-dimethyl-propylbromid und

- 3-(p-tert.-Butyl-phenyl)-1,2,3-trimethyl-propanol das 3-(p-tert.-Butyl-phenyl)-1,2,3-trimethyl-propylbromid.

42,2 g 2-Chlormethyl-2-phenyl-propan werden unter Stickstoffbegasung in 90 ml Dichlormethan gelöst, auf 0° gekühlt und 79,6 g Titantetrachlorid zugegeben. Anschliessend werden bei -5° innert 40 Minuten 28,7 g Dichlormethyl-methyläther zugetropft. Diese Lösung wird dann 2 Stunden bei 5-10° nachgerührt, auf 800 ml Eiswasser gegossen und das Produkt mit Aether extrahiert. Die vereinigten Aetherauszüge werden mit gesättigter Natriumbicarbonatlösung gewaschen, über Natriumsulfat getrocknet und eingeengt. Durch Destillation wird reiner p-(2-Chlor-1,1-dimethyl-äthyl)-benzaldehyd vom Siedepunkt 100-105°/0,04 Torr erhalten.

In analoger Weise erhält man ausgehend von:

- tert.-Amylbenzol und Dichlormethyl-methyläther den p-tert.-Amyl-benzaldehyd, Sdp. 65°/0,1 Torr,

- 1-Methyl-1-phenyl-cyclohexan und Dichlormethyl-methyläther den p-(1-Cyclohexyl-1-methyl)-benzaldehyd, Sdp. 94°/0,04 Torr,

- (2-Cyclohexyl-1,1-dimethyl-äthyl)-benzol und Dichlormethyl-methyläther den p-(2-Cyclohexyl-1,1-dimethyl-äthyl)-benzaldehyd, Sdp. 131-137°/0,05 Torr.

1. Vaginaltabletten

Beispiel 28

Vaginaltabletten enthaltend

| | | |
|---|---|---|
| Wirkstoff entsprechend Tabelle II | 100 mg | 50 mg |
| sek.-Calciumphosphat.$2H_2O$ | 300 mg | 400,0 mg |
| STA-RX 1500 (direkt pressbare Stärke) | 203 mg | 261,5 mg |
| Milchzucker (sprühgetrocknet) | 100 mg | 400,0 mg |
| Polyvinylpyrrolidon K 90 | 30 mg | 25,0 mg |
| Zitronensäure (wasserfrei) | 5 mg | 5,0 mg |
| Magnesiumstearat | 7 mg | 6,0 mg |
| | 745 mg | 695,0 mg |

2. Salben

Beispiel 29

Salbe für topische Applikation enthaltend

| | |
|---|---|
| Wirkstoff entsprechend Tabelle II | 1,00 g |
| Cetylalkohol | 3,60 g |
| Wollfett | 9,00 g |
| Vaseline weiss | 79,00 g |
| Paraffinöl | 7,40 g |
| | 100,00 g |

3. Crème

## Beispiel 30

Crème für topische Applikation enthaltend

| | | |
|---|---|---|
| Wirkstoff entsprechend Tabelle II | | 1,00 g |
| Polyoxyäthylenstearat (MYRJ 52) | | 3,00 g |
| Stearylalkohol | | 8,00 g |
| Paraffinöl int. dickflüssig | | 10,00 g |
| Vaseline weiss | | 10,00 g |
| CARBOPOL 934 Ph | | 0,30 g |
| NaOH reinst | | 0,07 g |
| Wasser entsalzt | ad | 100,00 g |

## Patentansprüche

1. Verbindungen der allgemeinen Formel

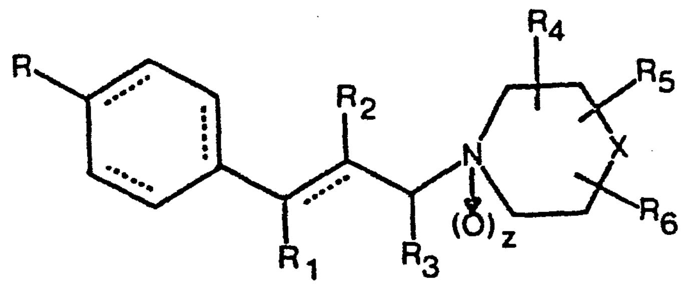

I

worin R Alkyl mit 4-12 Kohlenstoffatomen, wobei der Alkylrest gegebenenfalls mit Halogen substituiert sein kann; Cycloalkyl mit 3-7 Kohlenstoffatomen; Mono-nieder Alkyl substituiertes Cycloalkyl mit 4-10 Kohlenstoffatomen; Cycloalkylalkyl mit 4-16 Kohlenstoffatomen; Phenyl oder Aryl-nieder Alkyl mit 7-16 Kohlenstoffatomen; $R_1$ Wasserstoff, Hydroxy, Halogen, Alkoxy mit 1-4 Kohlenstoffatomen oder Alkyl mit 1-4 Kohlenstoffatomen; $R_2$ und $R_3$ Wasserstoff oder Alkyl mit 1-4 Kohlenstoffatomen; $R_4$, $R_5$ und $R_6$ Wasserstoff oder Alkyl mit 1-8 Kohlenstoffatomen, wobei zwei der Substituenten $R_4$, $R_5$ und $R_6$ jeweils mit dem gleichen Kohlenstoffatom verknüpft sein können oder zusammen einen ankondensierten alicyclischen oder aromatischen Sechsring bilden können; X eine Methylengruppe, ein Sauerstoffatom, eine Carbonylgruppe oder die Gruppe

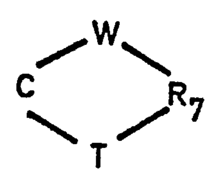

in welcher W und T jeweils für sich ein Sauerstoff- oder Schwefelatom und $R_7$ nieder Alkylen mit 2-4 Kohlenstoffatomen oder Alkenylen mit 4 Kohlenstoffatomen darstellen; oder die Gruppe

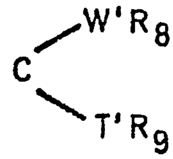

worin W' und T' ein Sauerstoff- oder Schwefelatom und $R_8$ und $R_9$ nieder Alkyl mit 1-6 Kohlenstoffatomen darstellen; Z die ganzen Zahlen O oder 1 bedeuten und die gestrichelten Bindungen hydriert sein können; mit der Massgabe, dass im Fall X in der Bedeutung einer Methylengruppe oder eines Sauerstoffatoms vorliegt, entweder der Substituent R lediglich einen Alkylrest mit 4-12 Kohlenstoffatomen bedeutet, der mit Halogen substituiert ist, oder der Substituent $R_1$ in der Bedeutung Hydroxy, Halogen oder Alkoxy mit 1-4 Kohlenstoffatomen vorliegt;

und Salze von solchen Verbindungen, die basischen Charakter aufweisen.

2. Verbindungen der allgemeinen Formel

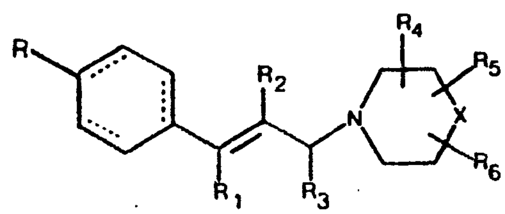

VIIa

worin R, $R_1$, $R_2$, $R_3$, $R_4$, $R_5$, $R_6$, X und die gestrichelten Bindungen die in Formel I angegebene Bedeutung besitzen.

3. Verbindungen der allgemeinen Formel

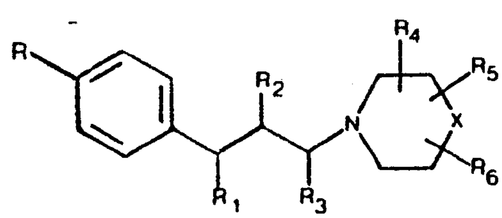

VIIb

worin R, $R_1$, $R_2$, $R_3$, $R_4$, $R_5$, $R_6$ und X die in Formel I angegebene Bedeutung besitzen.

4. Verbindungen der allgemeinen Formel

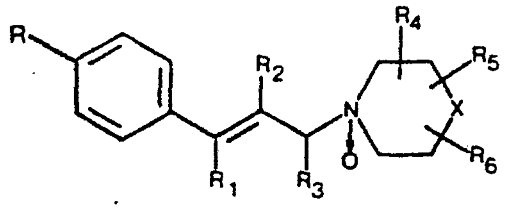

Ic

worin R, $R_1$, $R_2$, $R_3$, $R_4$, $R_5$, $R_6$ und X die in Formel I angegebene Bedeutung besitzen.

5. Verbindungen der allgemeinen Formel

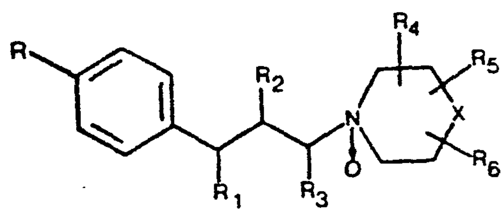

Ie

worin R, $R_1$, $R_2$, $R_3$, $R_4$, $R_5$, $R_6$ und X die in Formel I angegebene Bedeutung besitzen.

6. Verbindungen gemäss einem der Ansprüche 1-5, worin R in der Bedeutung 2-Chlor-1,1-dimethyl-äthyl, tert.-Butyl oder tert.-Amyl vorliegt.

7. 1-/3-[p-(2-Chlor-1,1-dimethyl-äthyl)-phenyl]-2-methyl-propyl/-piperidin; 1-/3-[p-(2-Chlor-1,1-dimethyl-äthyl)-phenyl]-2-methyl-propyl/-3,5-dimethyl-piperidin; 4-/3-[p-(2-Chlor-1,1-dimethyl-äthyl)-phenyl]-2-methyl-propyl/-2,6-dimethyl-morpholin; 1-/3-[p-(2-Chlor-1,1-dimethyl-äthyl)-phenyl]-2-methyl-propyl/-4,4-äthylendioxy-piperidin; 1-/3-[p-(2-Chlor-1,1-dimethyl-äthyl)-phenyl]-2-methyl-propyl/-4-piperidon; 1-/3-[p-(2-Chlor-1,1-dimethyl-

äthyl)-phenyl]-2,3-dimethyl-2-propenyl/-piperidin; 1-/3-[p-(2-Chlor-1,1-dimethyl-äthyl)-phenyl]-2,3-dimethyl-2-propenyl/-3,5-dimethyl-piperidin; 4-/3-[p-(2-Chlor-1,1-dimethyl-äthyl)-phenyl]-2,3-dimethyl-2-propenyl/-2,6-dimethyl-morpholin; 1-/3-[p-(2-Chlor-1,1-dimethyl-äthyl)-phenyl]-2,3-dimethyl-2-propenyl/-4,4-äthylendioxy-piperidin; 1-/3-[p-(2-Chlor-1,1-dimethyl-äthyl)-phenyl]-2,3-dimethyl-2-propenyl/-4-piperidon; 1-[3-(p-tert.-Butyl-phenyl)-3-hydroxy-2-methyl-propyl]-piperidin; 1-[3-(p-tert.-Butyl-phenyl)-3-methoxy-2-methyl-propyl]-piperidin; 1-[3-(p-tert.-Butyl-phenyl)-3-chlor-2-methyl-propyl]-piperidin; 1-[3-(p-tert.-Butyl-phenyl)-2-methyl-propyl]-4,4-äthylendioxy-piperidin; 1-[3-(p-tert.-Butyl-phenyl)-2-methyl-propyl]-3-methyl-4-piperidon; 1-[3-(p-tert.-Butyl-phenyl)-2-methyl-propyl]-3,5-dimethyl-4-piperidon; 1-[3-(p-tert.-Butyl-phenyl)-2-methyl-propyl]-4-piperidon; 1-[3-(p-tert.-Butyl-phenyl)-2-methyl-propyl]-4,4-äthylendioxy-3-methyl-piperidin; 1-[3-(p-tert.-Butyl-phenyl)-2-methyl-propyl]-4,4-dimethoxy-piperidin; 1-[3-(p-tert.-Butyl-phenyl)-2,3-dimethyl-2-propenyl]-4-piperidon; 1-[3-(p-tert.-Butyl-phenyl)-2,3-dimethyl-2-propenyl]-4,4-äthylendioxy-piperidin; 1-[3-(p-tert.-Amyl-phenyl)-2-methyl-propyl]-4,4-äthylendioxy-piperidin; 1-[3-(p-tert.-Amyl-phenyl)-2-methyl-propyl]-4-piperidon; 1-[3-(p-tert.-Amyl-phenyl)-2-methyl-propyl]-3-methyl-4-piperidon; 1-[3-(p-tert.-Amyl-phenyl)-2-methyl-propyl]-3,5-dimethyl-4-piperidon; 1-[3-(p-tert.-Amyl-phenyl)-2-methyl-propyl]-4,4-äthylendioxy-3-methyl-piperidin; 1-[3-(p-tert.-Amyl-phenyl)-2-methyl-propyl]-4,4-dimethoxy-piperidin; 1-[3-(p-tert.-Amyl-phenyl)-2,3-dimethyl-2-propenyl]-4,4-äthylendioxy-piperidin; 1-[3-(p-tert.-Amyl-phenyl)-2,3-dimethyl-2-propenyl]-4-piperidon; 1-/3-[p-(1-Cyclohexyl-1-methyl)-phenyl]-2-methyl-propyl/-4,4-äthylendioxy-piperidin; 1-/3-[p-(1-Cyclohexyl-1-methyl)-phenyl]-2-methyl-propyl/-4-piperidon; 1-/3-[p-(1-Cyclohexyl-1-methyl)-phenyl]-2,3-dimethyl-2-propenyl/-4,4-äthylendioxy-piperidin; 1-/3-[p-(1-Cyclohexyl-1-methyl)-phenyl]-2,3-dimethyl-2-propenyl/-4-piperidon.

8. Verbindungen der Ansprüche 1-7 als Fungicide.

9. Fungizides Mittel, dadurch gekennzeichnet, dass es eine wirksame Menge von mindestens einer Verbindung der allgemeinen Formel

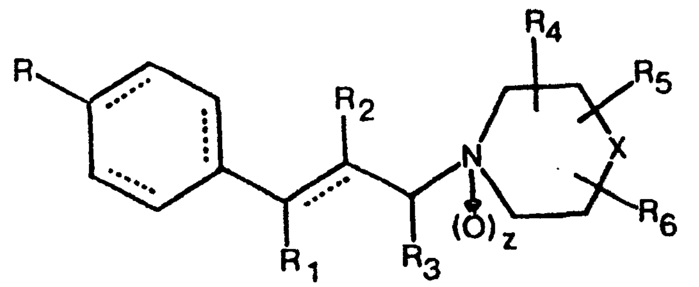

I

worin R Alkyl mit 4-12 Kohlenstoffatomen, wobei der Alkylrest gegebenenfalls mit Halogen substituiert sein kann; Cycloalkyl mit 3-7 Kohlenstoffatomen; Mono-nieder Alkyl substituiertes Cycloalkyl mit 4-10 Kohlenstoffatomen; Cycloalkylalkyl mit 4-16 Kohlenstoffatomen; Phenyl oder Aryl-nieder Alkyl mit 7-16 Kohlenstoffatomen; $R_1$ Wasserstoff, Hydroxy, Halogen, Alkoxy mit 1-4 Kohlenstoffatomen oder Alkyl mit 1-4 Kohlenstoffatomen; $R_2$ und $R_3$ Wasserstoff oder Alkyl mit 1-4 Kohlenstoffatomen; $R_4$, $R_5$ und $R_6$ Wasserstoff oder Alkyl mit 1-8 Kohlenstoffatomen, wobei zwei der Substituenten $R_4$, $R_5$ und $R_6$ jeweils mit dem gleichen Kohlenstoffatom verknüpft sein können oder zusammen einen ankondensierten alicyclischen oder aromatischen Sechsring bilden können; X eine Methylgruppe, ein Sauerstoffatom, eine Carbonylgruppe oder die Gruppe

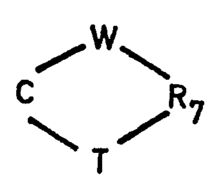

in welcher W und T jeweils für sich ein Sauerstoff- oder Schwefelatom und $R_7$ nieder Alkylen mit 2-4 Kohlenstoffatomen oder Alkenylen mit 4 Kohlenstoffatomen darstellen; oder die Gruppe

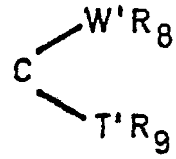

worin W' und T' ein Sauerstoff- oder Schwefelatom und $R_8$ und $R_9$ nieder Alkyl mit 1-6 Kohlenstoffatomen darstellen; Z die ganzen Zahlen 0 oder 1 bedeuten und die gestrichelten Bindungen hydriert sein können; mit der Massgabe, dass im Fall X in der Bedeutung einer Methylengruppe oder eines Sauerstoffatoms vorliegt, entweder der Substituent R lediglich einen Alkylrest mit 4-12 Kohlenstoffatomen bedeutet, der mit Halogen substituiert ist, oder der Substituent $R_1$ in der Bedeutung Hydroxy, Halogen oder Alkoxy mit 1-4 Kohlenstoffatomen vorliegt;

und Salze von solchen Verbindungen, die basischen Charakter aufweisen, und inertes Trägermaterial enthält.

10. Mittel gemäss Anspruch 9, dadurch gekennzeichnet, dass es eine wirksame Menge von mindestens einer Verbindung der Formel

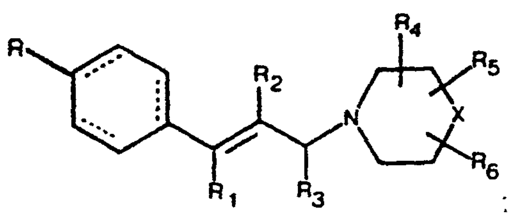

VIIa

worin R, $R_1$, $R_2$, $R_3$, $R_4$, $R_5$, $R_6$, X und die gestrichelten Bindungen die in Formel I angegebene Bedeutung besitzen,

enthält.

11. Mittel gemäss Anspruch 9, dadurch gekennzeichnet, dass es eine wirksame Menge von mindestens einer Verbindung der Formel

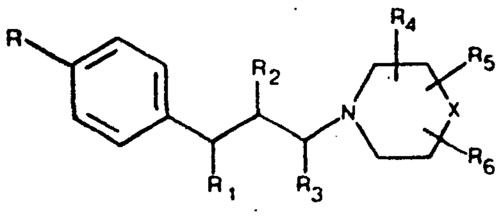

VIIb

worin R, $R_1$, $R_2$, $R_3$, $R_4$, $R_5$, $R_6$ und X die in Formel I angegebene Bedeutung besitzen,
enthält.

12. Mittel gemäss Anspruch 9, dadurch gekennzeichnet, dass es eine wirksame Menge von mindestens einer Verbindung der Formel

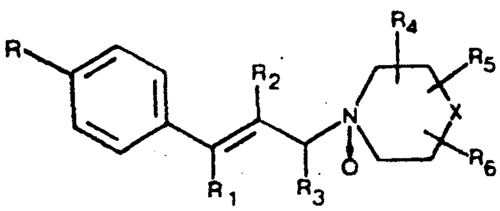

Ic

worin R, $R_1$, $R_2$, $R_3$, $R_4$, $R_5$, $R_6$ und X die in Formel I angegebene Bedeutung besitzen,
enthält.

13. Mittel gemäss Anspruch 9, dadurch gekennzeichnet, dass es eine wirksame Menge von mindestens einer Verbindung der Formel

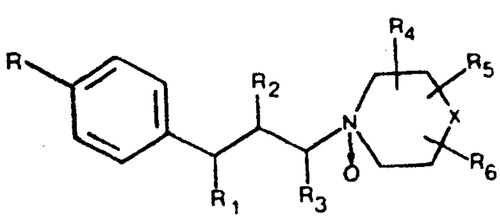

Ie

worin R, $R_1$, $R_2$, $R_3$, $R_4$, $R_5$, $R_6$ und X die in Formel I angegebene Bedeutung besitzen,
enthält.

14. Mittel gemäss Anspruch 9, dadurch gekennzeichnet, dass es eine Verbindung der Formel I, worin R in der Bedeutung 2-Chlor-1,1-dimethyläthyl, tert.-Butyl oder tert.-Amyl vorliegt, enthält.

15. Mittel gemäss einem der Ansprüche 9-14, dadurch gekennzeichnet, dass es 1-/3-[p-(2-Chlor-1,1-dimethyl-äthyl)-phenyl]-2-methyl-propyl/-piperidin; 1-/3-[p-(2-Chlor-1,1-dimethyl-äthyl)-phenyl]-2-methyl-propyl/-3,5-dimethyl-piperidin; 4-/3-[p-(2-Chlor-1,1-dimethyl-äthyl)-phenyl]-2-methyl-propyl/-2,6-dimethyl-morpholin; 1-/3-[p-(2-Chlor-1,1-dimethyl-äthyl)-phenyl]-2-methyl-propyl/-4,4-äthylendioxy-piperidin; 1-/3-[p-(2-Chlor-1,1-dimethyl-äthyl)-phenyl]-2-methyl-propyl/-4-piperidin; 1-/3-[p-(2-Chlor-1,1-dimethyl-äthyl)-phenyl]-2,3-dimethyl-2-propenyl/-piperidin; 1-/3-[p-(2-Chlor-1,1-dimethyl-äthyl)-phenyl]-2,3-dimethyl-2-propenyl/-3,5-dimethyl-piperidin; 4-/3-[p-(2-Chlor-1,1-dimethyl-äthyl)-phenyl]-2,3-dimethyl-2-propenyl/-2,6-dimethyl-morpholin; 1-/3-[p-(2-Chlor-1,1-dimethyl-äthyl)-phenyl]-2,3-dimethyl-2-propenyl/-4,4-äthylendioxy-piperidin; 1-/3-[p-(2-Chlor-1,1-dimethyl-äthyl)-phenyl]-2,3-dimethyl-2-propenyl/-4-piperidin; 1-[3-(p-tert.-Butyl-phenyl)-3-hydroxy-2-methyl-propyl]-piperidin; 1-[3-(p-tert.-Butyl-phenyl)-3-methoxy-2-methyl-propyl]-piperidin; 1-[3-(p-tert.-Butyl-phenyl)-3-chlor-2-methyl-propyl]-piperidin: 1-[3-(p-tert.-Butyl-phenyl)-2-methyl-propyl]-4,4-äthylendioxy-piperidin; 1-[3-(p-tert.-Butyl-phenyl)-2-methyl-propyl]-3-methyl-4-piperidon; 1-[3-(p-tert.-Butyl-phenyl)-2-methyl-propyl]-3,5-dimethyl-4-piperidon; 1-[3-(p-tert.-Butyl-phenyl)-2-methyl-propyl]-4-piperidon; 1-[3-(p-tert.-Butyl-phenyl)-2-methyl-propyl]-4,4-äthylendioxy-3-methyl-piperidin; 1-[3-(p-tert.-Butyl-phenyl)-2-methyl-propyl]-4,4-dimethoxy-piperidin; 1-[3-(p-tert.-Butyl-phenyl)-2,3-dimethyl-2-propenyl]-4-piperidon; 1-[3-(p-tert.-Butyl-phenyl)-2,3-dimethyl-2-propenyl]-4,4-äthylendioxy-piperidin; 1-[3-(p-tert.-Amyl-phenyl)-2-methyl-propyl]-4,4-äthylendioxy-piperidin; 1-[3-(p-tert.-

Amyl-phenyl)-2-methyl-propyl]-4-piperidon; 1-[3-(p-tert.-Amyl-phenyl)-2-methyl-propyl]-3-methyl-4-piperidon; 1-[3-(p-tert.-Amyl-phenyl)-2-methyl-propyl]-3,5-dimethyl-4-piperidon; 1-[3-(p-tert.-Amyl-phenyl)-2-methyl-propyl]-4,4-äthylendioxy-3-methyl-piperidin; 1-[3-(p-tert.-Amyl-phenyl)-2-methyl-propyl]-4,4-dimethoxy-piperidin; 1-[3-(p-tert.-Amyl-phenyl)-2,3-dimethyl-2-propenyl]-4,4-äthylendioxy-piperidin; 1-[3-(p-tert.-Amyl-phenyl)-2,3-dimethyl-2-propenyl]-4-piperidon; 1-/3-[p-(1-Cyclohexyl-1-methyl)-phenyl]-2-methyl-propyl/-4,4-äthylendioxy-piperidin; 1-/3-[p-(1-Cyclohexyl-1-methyl)-phenyl]-2-methyl-propyl/-4-piperidon; 1-/3-[p-(1-Cyclohexyl-1-methyl)-phenyl]-2,3-dimethyl-2-propenyl/-4,4-äthylendioxy-piperidin; 1-/3-[p-(1-Cyclohexyl-1-methyl)-phenyl]-2,3-dimethyl-2-propenyl/-4-piperidon enthält.

16. Verfahren zur Herstellung von Verbindungen der allgemeinen Formel

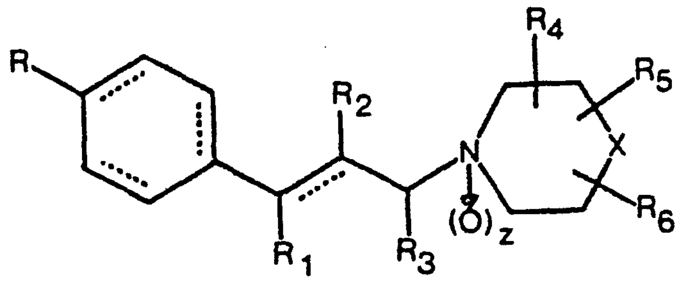

I

worin R Alkyl mit 4-12 Kohlenstoffatomen, wobei der Alkylrest gegebenenfalls mit Halogen substituiert sein kann; Cycloalkyl mit 3-7 Kohlenstoffatomen; Mono-nieder Alkyl substituiertes Cycloalkyl mit 4-10 Kohlenstoffatomen; Cycloalkylalkyl mit 4-16 Kohlenstoffatomen; Phenyl oder Aryl-nieder Alkyl mit 7-16 Kohlenstoffatomen; $R_1$ Wasserstoff, Hydroxy, Halogen, Alkoxy mit 1-4 Kohlenstoffatomen oder Alkyl mit 1-4 Kohlenstoffatomen; $R_2$ und $R_3$ Wasserstoff oder Alkyl mit 1-4 Kohlenstoffatomen; $R_4$, $R_5$ und $R_6$ Wasserstoff oder Alkyl mit 1-8 Kohlenstoffatomen, wobei zwei der Substituenten $R_4$, $R_5$ und $R_6$ jeweils mit dem gleichen Kohlenstoffatom verknüpft sein können oder zusammen einen ankondensierten alicyclischen oder aromatischen Sechsring bilden können; X eine Methylengruppe, ein Sauerstoffatom, eine Carbonylgruppe oder die Gruppe

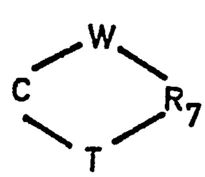

in welcher W und T jeweils für sich ein Sauerstoff- oder Schwefelatom und $R_7$ nieder Alkylen mit 2-4 Kohlenstoffatomen oder Alkenylen mit 4 Kohlenstoffatomen darstellen; oder die Gruppe

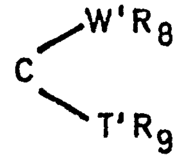

worin W' und T' ein Sauerstoff- oder Schwefelatom und $R_8$ und $R_9$ nieder Alkyl mit 1-6 Kohlenstoffatomen darstellen; Z die ganzen Zahlen 0 oder 1 bedeuten und die gestrichelten Bindungen hydriert sein können; mit der Massgabe, dass im Fall X in der Bedeutung einer Methylengruppe oder eines Sauerstoffatoms vorliegt, entweder der Substituent R lediglich einen Alkylrest mit 4-12 Kohlenstoffatomen bedeutet, der mit Halogen substituiert ist, oder der Substituent $R_1$ in der Bedeutung Hydroxy, Halogen oder Alkoxy mit 1-4 Kohlenstoffatomen vorliegt;

und Salze von solchen Verbindungen, die basischen Charakter aufweisen, dadurch gekennzeichnet, dass man

a) ein Halogenid der Formel

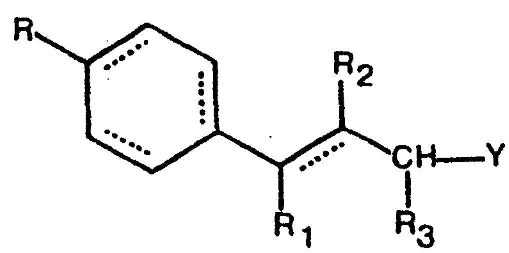

II

worin R, $R_1$, $R_2$, $R_3$ und die gestrichelten Bindungen die in Formel I angegebene Bedeutung besitzen und Y Chlor, Brom oder Jod bedeutet,

mit einer Verbindung der Formel

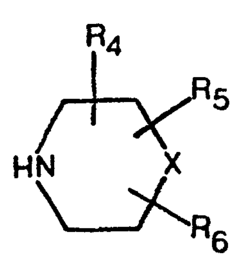

III

worin $R_4$, $R_5$, $R_6$ und X, die in Formel I angegebene Bedeutung besitzen,

umsetzt, oder

b) in einer Verbindung der Formel

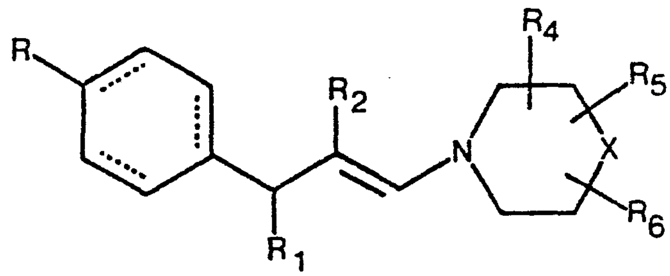

IV

worin R, $R_1$, $R_2$, $R_4$, $R_5$, $R_6$ und X und die gestrichelten Bindungen die in Formel I angegebene Bedeutung besitzen,

die aliphatische Doppelbindung katalytisch hydriert oder mit Ameisensäure reduziert,

c) eine Verbindung der Formel

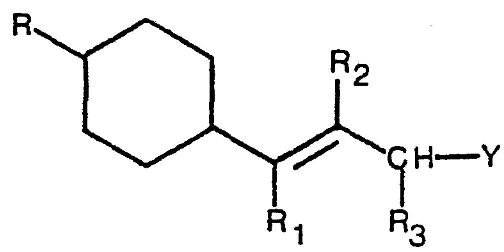

V

worin R, $R_1$, $R_2$, $R_3$ und Y die in Formel II angegebene Bedeutung besitzen,

mit einer Verbindung der Formel III umsetzt, oder

d) eine Verbindung der Formel

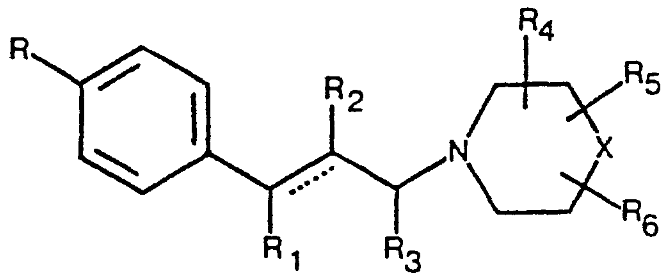

VI

worin R, $R_1$, $R_2$, $R_3$, $R_4$, $R_5$, $R_6$ und X und die gestrichelte Bindung die in Formel I angegebene Bedeutung besitzen,

katalytisch hydriert, oder

e) eine Verbindung der Formel

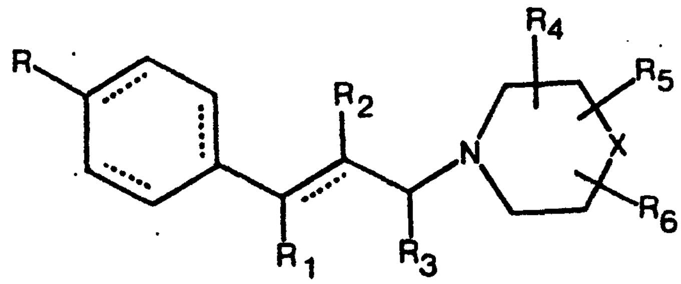

VII

worin R, $R_1$, $R_2$, $R_3$, $R_4$, $R_5$, $R_6$ und X und die gestrichelten Bindungen die in Formel I angegebene Bedeutung besitzen,

mit Wasserstoffperoxid oder Persäuren behandelt, oder

f) eine Verbindung der Formel VII, worin X die Gruppe

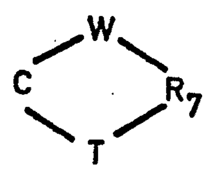

oder die Gruppe

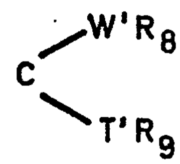

wobei in diesen Gruppen die Symbole W, W', T, T', $R_7$, $R_8$ und $R_9$ die in Formel I angegebene Bedeutung besitzen,

bedeutet, in Gegenwart eines sauren Mittels zu einer Verbindung der Formel VII, worin X eine Carbonylgruppe bedeutet, in an sich bekannter Weise hydrolysiert, oder

g) eine Verbindung der Formel VII, worin X eine Carbonylgruppe bedeutet, in an sich bekannter Weise in die entsprechenden Ketale, Mercaptole, Thioketale oder Hemithioketale der Formel VII, worin X die Gruppe

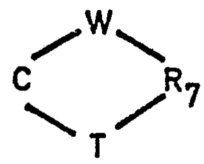

oder die Gruppe

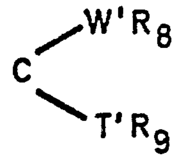

wobei in diesen Gruppen die Symbole W, W', T, T', $R_7$, $R_8$ und $R_9$ die in Formel I angegebene Bedeutung besitzen,

bedeutet, in Gegenwart eines sauren Mittels überführt, oder

h) eine Verbindung der Formel I, die basischen Charakter besitzt, mit einer Säure in an sich bekannter Weise in ein Salz überführt.

17. Verfahren nach Anspruch 1a zur Herstellung einer Verbindung der Formel

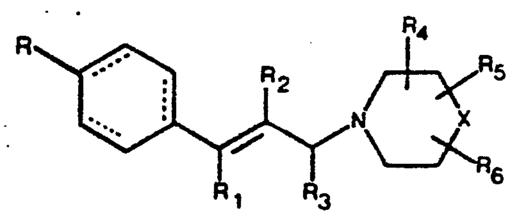

VIIa

worin R, $R_1$, $R_2$, $R_3$, $R_4$, $R_5$, $R_6$, X und die gestrichelten Bindungen die in Formel I angegebene Bedeutung haben,

dadurch gekennzeichnet, dass man eine Verbindung der Formel

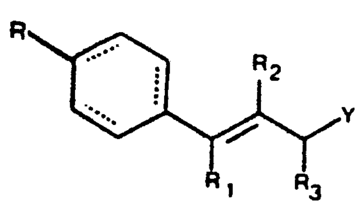

IIa

worin R, $R_1$, $R_2$, $R_3$ und Y die in Formel II angegebene Bedeutung besitzen,
in Diäthyläther bei einer Temperatur zwischen $0^oC$ und der Rückflusstemperatur des Reaktionsgemisches umsetzt.

18. Verfahren nach Anspruch 1a zur Herstellung einer Verbindung der Formel

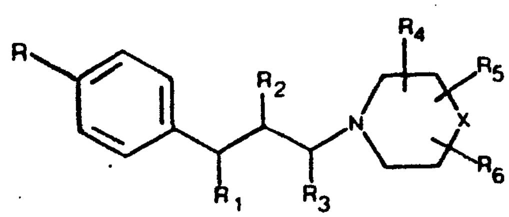

VIIb

worin R, $R_1$, $R_2$, $R_3$, $R_4$, $R_5$, $R_6$ und X die in Formel I angegebene Bedeutung haben,
dadurch gekennzeichnet, dass man eine Verbindung der Formel

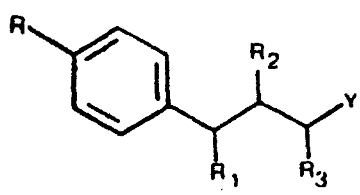

IIb

worin R, $R_1$, $R_2$, $R_3$ und Y die in Formel II angegebene Bedeutung besitzen,
in Aethylenglykol oder Glycerin in einem Temperaturbereich zwischen $50^oC$ und $150^oC$ umsetzt.

19. Verfahren zur Bekämpfung von Pflanzenfungi, dadurch gekennzeichnet, dass man ein Mittel gemäss den Ansprüchen 9-15 auf die zu schützenden Gegenstände in wirksamen Mengen appliziert.

20. Verwendung eines Mittels gemäss den Ansprüchen 9-15 zur Bekämpfung von Pflanzenfungi.

***